(19) Europäisches Patentamt
European Patent Office
Office européen des brevets

(11) **EP 3 316 837 B1**

(12) **EUROPEAN PATENT SPECIFICATION**

(45) Date of publication and mention
of the grant of the patent:
**08.12.2021 Bulletin 2021/49**

(51) Int Cl.:
*A61F 13/511* (2006.01)      *D04H 1/54* (2012.01)
*D04H 3/16* (2006.01)

(21) Application number: **16738961.8**

(86) International application number:
**PCT/US2016/040251**

(22) Date of filing: **30.06.2016**

(87) International publication number:
**WO 2017/004302 (05.01.2017 Gazette 2017/01)**

(54) **NONWOVEN WEB FORMED WITH LOFT-ENHANCING CALENDER BOND SHAPES AND PATTERNS, AND ARTICLES INCLUDING THE SAME**

VLIESBAHN MIT FLORVERSTÄRKENDEN KALANDERVERBINDUNGSFORMEN UND -MUSTERN UND ARTIKEL DAMIT

VOILE NON-TISSÉ FORMÉ DE FORMES ET DE MOTIFS DE LIAISON DE CALANDRE AMÉLIORANT LE GONFLANT, ET ARTICLES COMPRENANT CELUI-CI

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.06.2015 US 201514755810**

(43) Date of publication of application:
**09.05.2018 Bulletin 2018/19**

(73) Proprietor: **The Procter & Gamble Company Cincinnati, OH 45202 (US)**

(72) Inventors:
• **XU, Han**
**Cincinnati, Ohio 45202 (US)**
• **KEARNEY, Donald, R.**
**Cincinnati, Ohio 45202 (US)**
• **ISELE, Olaf, Erik Alexander**
**Cincinnati, Ohio 45202 (US)**
• **DEBEER, Antonius, Lambertus**
**Cincinnati, Ohio 45202 (US)**

(74) Representative: **P&G Patent Germany Procter & Gamble Service GmbH Sulzbacher Straße 40 65824 Schwalbach am Taunus (DE)**

(56) References cited:
**WO-A1-2012/134988      WO-A1-2014/022362**

**Description**

BACKGROUND OF THE INVENTION

The business of manufacturing and marketing disposable absorbent articles for personal care or hygiene (such as disposable diapers, training pants, adult incontinence undergarments, feminine hygiene products, breast pads, care mats, bibs, wound dressing products, and the like) is relatively capital intensive and highly competitive. To maintain or grow their market share and thereby maintain a successful business, manufacturers of such articles must continually strive to enhance their products in ways that serve to differentiate them from those of their competitors, while at the same time controlling costs so as to enable competitive pricing and the offering to the market of an attractive value-to-price proposition.

One way in which some manufacturers may seek to enhance such products is through enhancements to softness. Parents and caregivers naturally seek to provide as much comfort as they can for their babies, and utilizing products such as disposable diapers that they perceive as relatively soft provides reassurance that they are doing what they can to provide comfort in that context. With respect to other types of disposable absorbent articles that are designed to be applied and/or worn close to the skin, an appearance of softness can reassure the wearer or caregiver that the article will be comfortable.

Thus, manufacturers may devote efforts toward enhancing the softness of the various materials used to make such products, such as various web materials, including nonwoven web materials formed from polymer fibers, and laminates thereof, forming the products. Such laminates may include, for example, laminates of polymer films and nonwoven web materials forming the backsheet components of the products.

It is believed that humans' perceptions of softness of a nonwoven web material can be affected by tactile signals, auditory signals and visual signals.

Tactile softness signals may be affected by a variety of the material's features and properties that have effect on its tactile feel, among them volume density, basis weight, pliability and flexibility of the nonwoven web, and loft or caliper.

It is believed that perceptions of softness of a material also may be affected by visual signals, *i.e.,* its visual appearance. It is believed that, if a nonwoven material *looks* relatively soft to a person, it is much more likely that the person will perceive it as having relative tactile softness as well. Visual impressions of softness may be affected by a variety of features and properties, including but not limited to color, opacity, light reflectivity, refractivity or absorption, macroscopic physical surface features, and again, loft or caliper.

Loft or caliper in nonwovens may have importance for reasons in addition to or other than creating an impression of softness. In some applications, nonwovens may be used as components of cleaning articles, such as wipes or dusters. Improving loft of such a nonwoven can also improve its efficacy as a cleaning element. In another particular application, a nonwoven may be used to form the loops component of a hook-and-loop fastening system. Improving loft of such a nonwoven can improve its suitability for this purpose.

Various efforts have been made to provide or alter features of nonwoven web materials with the objective of enhancing loft and/or consumer perceptions of softness. These efforts have included selection and/or manipulation of fiber chemistry, basis weight, fiber density, configuration and size, tinting and/or opacifying, embossing or bonding in various patterns, etc.

For example, in U.S. 2013/0253461A1, features of a bonding pattern, reflecting corresponding features of a calender bonding roller (bonding protrusions and the configurations thereof on the roller), are disclosed. It is believed that the disclosed features, in various combinations, advantageously promote and affect airflow through the nip between the bonding roller and mating anvil roller in a way that causes air to tease or fluff fibers of the nonwoven as it exits the nip, enhancing the loft of the nonwoven product.

The approach described above, however, has not fully addressed all needs. In one particular, it would be advantageous if the loft-enhancing features of the bonding roller described in the cited reference could be enjoyed, while providing further enhancements to mechanical strength (machine direction and cross direction tensile strength) and dimensional stability (resistance to neckdown) of the nonwoven web product.

The challenge to improve loft while preserving or even improving mechanical strength and dimensional stability becomes more difficult as nonwoven web basis weight is reduced, because, as basis weight is reduced, fewer fibers per unit surface area are available to contribute to loft and strength of the web.

WO 2014/022362 relates to a diaper structure with enhanced softness attributes. The diaper structure may include an innermost layer formed of a first nonwoven web, and an outermost layer formed of a second nonwoven web. The first and second nonwoven webs may have a combined basis weight of at least about 30 gsm. Characteristics affecting pressure diffusion at tactile pressure points may be balanced within ranges between the first and second webs to provide similar tactile pressure diffusion characteristics for the inside and outside of the diaper.

BRIEF DESCRIPTION OF THE DRAWINGS

Fig. 1A is a perspective view of a disposable diaper shown laid out horizontally in a relaxed condition, wearer-facing surfaces up;

Fig. 1B is a plan view of a disposable diaper shown laid out horizontally in a stretched out, flattened state (stretched out against elastic contraction induced by the presence of elastic members), wearer-facing surfaces facing the viewer;

Fig. 2A is a cross section of the diaper depicted in Figs. 1A and 1B, taken through line 2-2 in those figures;

Fig. 2B is a schematic cross section of a portion of a laminate of a polymeric film and a nonwoven web, taken through a pattern of bond impressions in the nonwoven web;

Fig. 3 is a simplified schematic view of a batt moving through the nip between calender rollers to form a calender-bonded nonwoven web;

Fig. 4A is a plan view of a prior art view of a pattern of bonding surface shapes of bonding protrusions that may be imparted to the surface of a calender roller, to create a corresponding pattern of consolidating bond impressions having bond shapes in a nonwoven web;

Fig. 4B is an enlarged, cross-section view of a bonding protrusion;

Fig. 5A is a plan view of a pattern of bonding surface shapes of bonding protrusions that may be imparted to the surface of a calender roller, to create a corresponding pattern of consolidating bond impressions having bond shapes in a nonwoven web, within the scope of the present disclosure;

Figs. 5B-5D are various plan views of portions of the pattern depicted in Fig. 5A, expanded to facilitate descriptions of various details and features of the pattern; and

Fig. 5E is a plan view of another example of a pattern of bonding surface shapes of bonding protrusions that may be imparted to the surface of a calender roller, to create a corresponding pattern of consolidating bond impressions having bond shapes in a nonwoven web, within the scope of the present disclosure.

DETAILED DESCRIPTION OF EXAMPLES

Definitions

"Absorbent article" refers to devices that absorb and contain body exudates, and, more specifically, refers to devices that are placed against or in proximity to the body of the wearer to absorb and contain the various exudates discharged from the body. Absorbent articles may include diapers, training pants, adult incontinence undergarments and pads, feminine hygiene pads, breast pads, care mats, bibs, wound dressing products, and the like. As used herein, the term "exudates" includes, but is not limited to, urine, blood, vaginal discharges, breast milk, sweat and fecal matter.

"Absorbent core" means a structure typically disposed between a topsheet and backsheet of an absorbent article for absorbing and containing liquid received by the absorbent article. The absorbent core may also include a cover layer or envelope. The cover layer or envelope may comprise a nonwoven. In some examples, the absorbent core may include one or more substrates, an absorbent polymer material, and a thermoplastic adhesive material/composition adhering and immobilizing the absorbent polymer material to a substrate, and optionally a cover layer or envelope.

"Absorbent polymer material," "absorbent gelling material," "AGM," "superabsorbent," and "superabsorbent material" are used herein interchangeably and refer to cross linked polymeric materials that can absorb at least 5 times their weight of an aqueous 0.9% saline solution as measured using the Centrifuge Retention Capacity test (Edana 441.2-01).

"Absorbent particulate polymer material" is used herein to refer to an absorbent polymer material which is in particulate form so as to be flowable in the dry state.

"Absorbent particulate polymer material area" as used herein refers to the area of the core wherein the first substrate and second substrate are separated by a multiplicity of superabsorbent particles. There may be some extraneous superabsorbent particles outside of this area between the first substrate 64 and second substrate.

"Airfelt" is used herein to refer to comminuted wood pulp, which is a form of cellulosic fiber.

A "batt" is used herein to refer to fiber materials prior to being consolidated in a final calendering process as described herein. A "batt" comprises individual fibers, which are usually unbonded to each other, although a certain amount of pre-bonding between fibers may be performed and is also included in the meaning, such as may occur during or shortly after the lay-down of fibers in a spunlaying process, or as may be achieved be a pre-calendering. This pre-bonding, however, still permits a substantial number of the fibers to be freely moveable such that they can be repositioned. A "batt" may comprise several strata, such as may result from depositing fibers from several beams in a spunlaying process.

"Bicomponent" refers to fiber having a cross-section comprising two discrete polymer components, two discrete blends of polymer components, or one discrete polymer component and one discrete blend of polymer components. "Bicomponent fiber" is encompassed within the term "multicomponent fiber." A Bicomponent fiber may have an overall cross section divided into two or more subsections of the differing components of any shape or arrangement, including, for example, coaxial subsections, core-and-sheath subsections, side-by-side subsections, radial subsections, etc.

"Bond area percentage" on a nonwoven web is a ratio of area occupied by bond impressions, to the total surface area of the web, expressed as a percentage, and measured according to the Bond Area Percentage Method set forth herein.

"Bonding roller," "calender roller" and "roller" are used interchangeably.

A "bond impression" in a nonwoven web is the surface structure created by the impression of a bonding protrusion on a calender roller into a nonwoven web. A bond impression is a location of deformed, intermeshed or entangled, and melted or thermally fused, materials from fibers superimposed and compressed in a z-direction beneath the bonding protrusion, which form a bond. The individual bonds may be connected in the nonwoven structure by loose fibres between them. The shape and size of the bond impression approximately corresponds to the shape and size of the bonding surface of a bonding protrusion on the calender roller.

A "column" of bonds on a nonwoven web is a group of bonds of like shape and rotational orientation that are arranged in regularly repeating configuration along a line that extends most predominately in the machine direction.

"Cross direction"(CD) - with respect to the making of a nonwoven web material and the nonwoven web material, refers to the direction along the web material substantially perpendicular to the direction of forward travel of the web material through the manufacturing line in which the web material is manufactured. With respect to a batt moving through the nip of a pair of calender rollers to form a bonded nonwoven web, the cross direction is perpendicular to the direction of movement through the nip, and parallel to the nip.

"Disposable" is used in its ordinary sense to mean an article that is disposed or discarded after a limited number of usage events over varying lengths of time, for example, fewer than about 20 events, fewer than about 10 events, fewer than about 5 events, or fewer than about 2 events.

"Diaper" refers to an absorbent article generally worn by infants and incontinent persons about the lower torso so as to encircle the waist and legs of the wearer and that is specifically adapted to receive and contain urinary and fecal waste. As used herein, term "diaper" also includes "pant" which is defined below.

"Fiber" and "filament" are used interchangeably.

"Fiber diameter" reflects the diameter or width of a fiber and is typically expressed in microns ($\mu$m). The terms "denier" ("den") and "decitex" ("dTex") are units that are alternatively used to characterize the fineness or coarseness of a fiber. 1 denier (or den) equals 1 gram of fiber per 9000 m. 1 decitex (or dTex) equals 1 gram per 10000 m. Denier and decitex relate to the fiber diameter and the mass/volume density of the material(s) of which the fiber is formed.

"Film" - means a skin-like or membrane-like layer of material formed of one or more polymers, which does not have a form consisting predominately of a web-like structure of consolidated polymer fibers and/or other fibers.

"Length" or a form thereof, with respect to a diaper or training pant, refers to a dimension measured along a direction perpendicular to the waist edges and/or parallel to the longitudinal axis.

"Machine direction" (MD) - with respect to the making of a nonwoven web material and the nonwoven web material, refers to the direction along the web material substantially parallel to the direction of forward travel of the web material through the manufacturing line in which the web material is manufactured. With respect to a nonwoven batt moving through the nip of a pair of calender rollers to form a bonded nonwoven web, the machine direction is parallel to the direction of movement through the nip, and perpendicular to the nip.

"Machine direction bias," with respect to fibers forming a nonwoven, means that the fibers' lengths have machine direction vector components that are greater than their cross direction vector components.

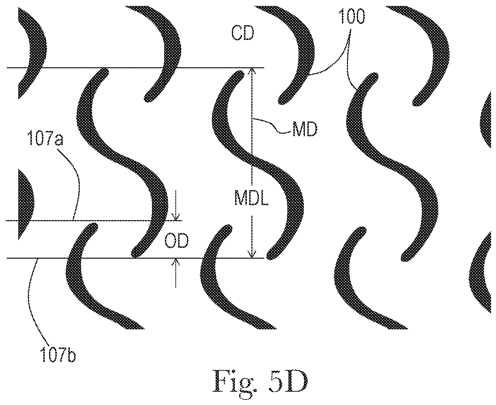

"Machine direction row overlap," with respect to two adjacent bonding shapes in successive rows, means that a line along the cross direction is tangent to or crosses the perimeters of both shapes. The machine direction row overlap is measured as the distance OD between a first cross direction line 107a tangent to the forward-most edge of the shape in the trailing row, and a second cross direction line 107b tangent to the rearward-most edge of the shape in the leading row; *see, e.g.,* Fig. 5D. The extent of machine direction overlap is expressed as ratio of distance OD to the machine direction length MDL of the shape in the leading row, or OD/MDL, times 100%. "Monocomponent" refers to fiber formed of a single polymer component or single blend of polymer components, as distinguished from bicomponent or multicomponent fiber.

"Multicomponent" refers to fiber having a cross-section comprising more than one discrete polymer component, more than one discrete blend of polymer components, or at least one discrete polymer component and at least one discrete blend of polymer components. "Multicomponent fiber" includes, but is not limited to, "bicomponent fiber." A multicomponent fiber may have an overall cross section divided into subsections of the differing components of any shape or arrangement, including, for example, coaxial subsections, core-and-sheath subsections, side-by-side subsections, radial subsections, islands-in-the-sea, etc.

"Neckdown" refers to the tendency of a web material to exhibit a reduction in cross-direction width when strained in the machine direction, a Poisson effect-like behavior.

A "nonwoven" is a manufactured sheet or web of directionally or randomly oriented fibers which are first formed into a batt and then consolidated and bonded together by friction, cohesion, adhesion or one or more patterns of bonds and bond impressions created through localized compression and/or application of pressure, heat, ultrasonic or heating energy, or a combination thereof. The term does not include fabrics which are woven, knitted, or stitch-bonded with yarns or filaments. The fibers may be of natural or man-made origin and may be staple or continuous filaments or be formed in situ. Commercially available fibers have diameters ranging from less than about 0.001 mm (1 $\mu$m) to more than about 0.2 mm (200 $\mu$m) and they come in several different forms: short fibers (known as staple, or chopped), continuous single fibers (filaments or monofilaments), untwisted bundles of continuous filaments (tow), and twisted bundles of continuous filaments (yarn). Nonwoven fabrics can be formed by many processes including but not limited to meltblowing, spunbonding, spunmelting, solvent spinning, electro spinning, carding, film fibrillation, melt-film fibrillation, airlaying, dry-laying, wetlaying with staple fibers, and combinations of these processes as known in the art. The basis weight of nonwoven fabrics is usually expressed in grams per square meter (gsm).

"Opacity" is a numeric value relating to the ability of a web material to transmit light therethrough, measured according the Opacity Measurement Method set forth herein.

"Pant" or "training pant", as used herein, refer to disposable garments having a waist opening and leg openings designed for infant or adult wearers. A pant may be placed in position on the wearer by inserting the wearer's legs into the leg openings and sliding the pant into position about a wearer's lower torso. A pant may be preformed by any suitable technique including, but not limited to, joining together portions of the article using refastenable and/or non-refastenable bonds (e.g., seam, weld, adhesive, cohesive bond, fastener, etc.). A pant may be preformed anywhere along the circumference of the article (*e.g.,* side fastened, front waist fastened). While the terms "pant" or "pants" are used herein, pants are also commonly referred to as "closed diapers," "prefastened diapers," "pull-on diapers," "training pants," and "diaper-pants". Suitable pants are disclosed in U.S. Pat. No. 5,246,433, issued to Hasse et al. on September 21, 1993; U.S. Pat. No. 5,569,234, issued to Buell et al. on October 29, 1996; U.S. Pat. No. 6,120,487, issued to Ashton on September 19, 2000; U.S. Pat. No. 6,120,489, issued to Johnson et al. on September 19, 2000; U.S. Pat. No. 4,940,464, issued to Van Gompel et al. on July 10, 1990; U.S. Pat. No. 5,092,861, issued to Nomura et al. on March 3, 1992; U.S. Patent Publication No. 2003/0233082 A1, entitled "Highly Flexible And Low Deformation Fastening Device", filed on June 13, 2002; U.S. Pat. No. 5,897,545, issued to Kline et al. on April 27, 1999; U.S. Pat. No. 5,957,908, issued to Kline et al. on September 28, 1999.

When used as an adjective in connection with a component of a material, the term "predominately" means that the component makes up greater than 50% by weight of the material. When used as an adjective in connection with a directional orientation of a physical feature or geometric attribute thereof, "predominately" means the feature or attribute has a projection onto a line extending along the direction indicated, greater in length than the projection onto a line perpendicular thereto. Within other contexts, the term "predominantly" refers to a condition which imparts a substantial effect on a property or feature. Thus, when a material comprises "predominantly" a component said to impart a property, this component imparts a property that the material otherwise would not exhibit. For example, if a material comprises "predominantly" heat-fusible fibers, the quantity and components of these fibers must be sufficient to allow heat fusion of the fibers.

A "bonding protrusion" or "protrusion" is a feature of a bonding roller at its radially outermost portion, surrounded by recessed areas. Relative the rotational axis of the bonding roller, a bonding protrusion has a radially outermost bonding surface with a bonding surface shape and a bonding surface shape area, which generally lies along an outer cylindrical surface with a substantially constant radius from the bonding roller rotational axis; however, protrusions having bonding surfaces of discrete and separate shapes are often small enough relative the radius of the bonding roller that the bonding surface may appear flat/planar; and the bonding surface shape area is closely approximated by a planar area of the same shape. A bonding protrusion may have sides that are perpendicular to the bonding surface, although usually the sides have an angled slope, such that the cross section of the base of a bonding protrusion is larger than its bonding surface. A plurality of bonding protrusions may be arranged on a calender roller in a pattern. The plurality of bonding protrusions has a bonding area per unit surface area of the outer cylindrical surface which can be expressed as a percentage, and is the ratio of the combined total of the bonding shape areas of the protrusions within the unit, to the total surface area of the unit.

A "row" of bonds on a nonwoven web is a group of bonds of like shape and rotational orientation that are arranged in regularly repeating configuration along a line that extends most predominately in the cross direction.

"Tensile Strength" refers to the maximum tensile force (Peak Force) a material will sustain before tensile failure, as measured by the Tensile Strength Measurement Method set forth herein.

"Thickness" and "caliper" are used herein interchangeably.

"Total Stiffness" refers to the measured and calculated value relating to a material, according to the Stiffness measurement method set forth herein.

"Volume mass" is the ratio of basis weight and caliper and indicates the bulkiness and fluffiness of the product, which are important properties of the nonwoven web according to the invention. The lower the value, the bulkier is the web.

$$\text{Volume mass (kg/m}^3) = \text{basis weight (g/m}^2) / \text{caliper (mm)}.$$

"Width" or a form thereof, with respect to a diaper or training pant, refers to a dimension measured along a direction parallel to the waist edges and/or perpendicular to the longitudinal axis.

"Z-direction," with respect to a web, means generally orthogonal or perpendicular to the plane approximated by the web along the machine and cross direction dimensions.

Examples of the present invention include disposable absorbent articles having improved softness attributes.

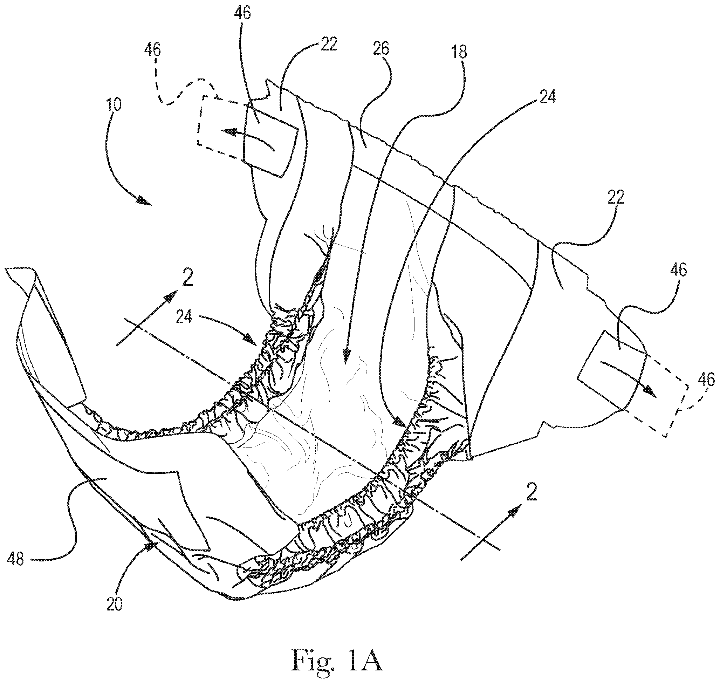

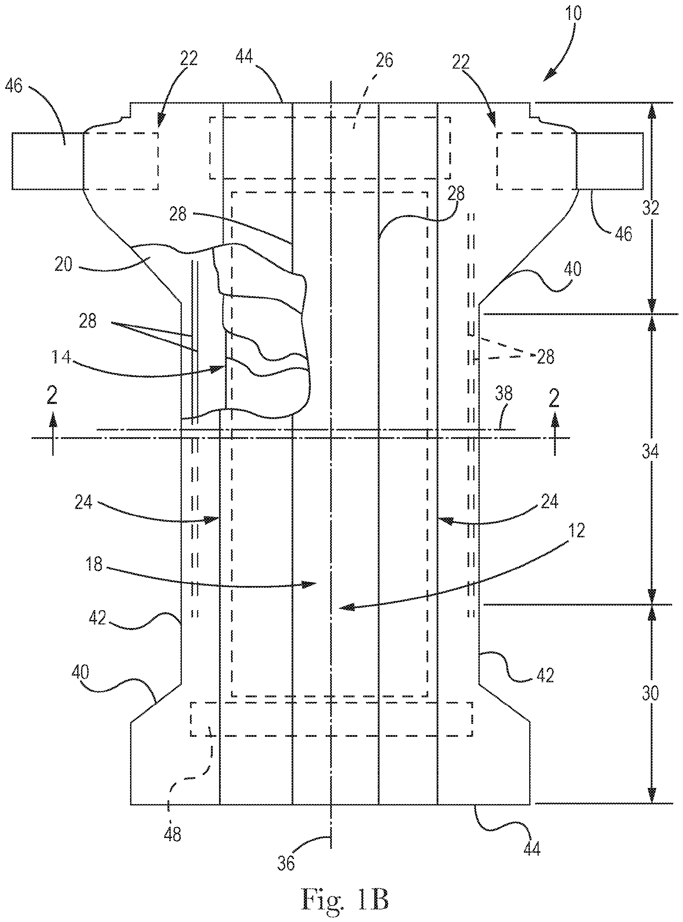

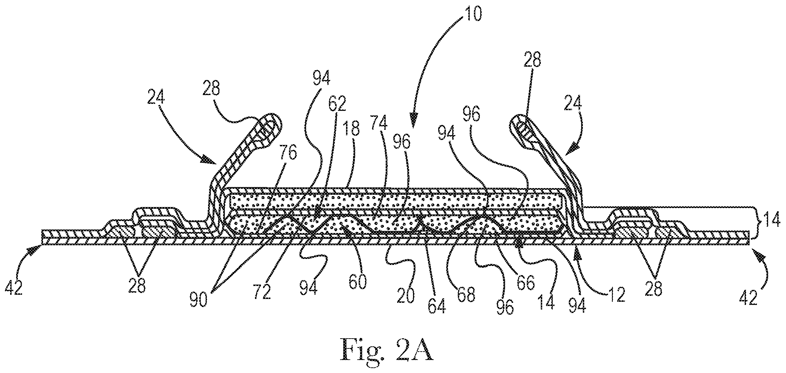

Fig. 1A is a perspective view of a diaper 10 in a relaxed, laid-open position as it might appear opened and lying on a horizontal surface. Fig. 1B is a plan view of a diaper 10 shown in a flat-out, uncontracted state (i.e., without elastic induced contraction), shown with portions of the diaper 10 cut away to show underlying structure. The diaper 10 is depicted in Fig. 1B with its longitudinal axis 36 and its lateral axis 38. Portions of the diaper 10 that contact a wearer are shown oriented upwards in Fig. 1A, and are shown facing the viewer in Fig. 1B. Fig. 2A is a cross section of the diaper taken at line 2-2 in Fig. 1B.

The diaper 10 generally may comprise a chassis 12 and an absorbent core 14 disposed in the chassis. The chassis 12 may comprise the main body of the diaper 10.

The chassis 12 may include a topsheet 18, which may be liquid pervious, and a backsheet 20, which may be liquid impervious. The absorbent core 14 may be encased between the topsheet 18 and the backsheet 20. The chassis 12 may also include side panels 22, elasticized leg cuffs 24, and an elastic waist feature 26. The chassis 12 may also comprise a fastening system, which may include at least one fastening member 46 and at least one landing zone 48. One or more layers of the topsheet and/or backsheet may be formed of a nonwoven web as described below.

The leg cuffs 24 and the elastic waist feature 26 may each typically comprise elastic members 28. One end portion of the diaper 10 may be configured as a first waist region 30 of the diaper 10. An opposite end portion of the diaper 10 may be configured as a second waist region 32 of the diaper 10. An intermediate portion of the diaper 10 may be configured as a crotch region 34, which extends longitudinally between the first and second waist regions 30 and 32. The crotch region 34 may include from 33.3% to 50% of the overall length of the diaper 10, and each of waist regions 30, 32 may correspondingly include from 25% to 33.3% of the overall length of the diaper 10.

The waist regions 30 and 32 may include elastic elements such that they gather about the waist of the wearer to provide improved fit and containment (elastic waist feature 26). The crotch region 34 is that portion of the diaper 10 which, when the diaper 10 is worn, is generally positioned between the wearer's legs.

The diaper 10 may also include such other features including front and rear ear panels, waist cap features, elastics and the like to provide better fit, containment and aesthetic characteristics. Such additional features are described in, e.g., U.S. Pats. Nos. 3,860,003 and 5,151,092.

In order to apply and keep diaper 10 in place about a wearer, the second waist region 32 may be attached by the fastening member 46 to the first waist region 30 to form leg opening(s) and an article waist. When fastened, the fastening system carries a tensile load around the article waist.

According to some examples, the diaper 10 may be provided with a re-closable fastening system or may alternatively be provided in the form of a pant-type diaper. When the absorbent article is a diaper, it may comprise a re-closable fastening system joined to the chassis for securing the diaper to a wearer. When the absorbent article is a pant-type diaper, the article may comprise at least two side panels joined to the chassis and to each other to form a pant. The fastening system and any component thereof may include any material suitable for such a use, including but not limited to plastics, films, foams, nonwoven, woven, paper, laminates, stretch laminates, activated stretch laminates, fiber reinforced plastics and the like, or combinations thereof. In some examples, the materials making up the fastening device may be flexible. In some examples, the fastening device may comprise cotton or cotton-like materials for additional softness or consumer perception of softness. The flexibility may allow the fastening system to conform to the shape of the body and thus, reduce the likelihood that the fastening system will irritate or injure the wearer's skin.

For unitary absorbent articles, the chassis 12 and absorbent core 14 may form the main structure of the diaper 10 with other features added to form the composite diaper structure. While the topsheet 18, the backsheet 20, and the absorbent core 14 may be assembled in a variety of well-known configurations, preferred diaper configurations are described generally in U.S. Pat. No. 5,554,145 entitled "Absorbent Article With Multiple Zone Structural Elastic-Like Film Web Extensible Waist Feature" issued to Roe et al. on Sep. 10, 1996; U.S. Pat. No. 5,569,234 entitled "Disposable Pull-On Pant" issued to Buell et al. on Oct. 29, 1996; and U.S. Pat. No. 6,004,306 entitled "Absorbent Article With Multi-Directional Extensible Side Panels" issued to Robles et al. on Dec. 21, 1999.

The topsheet 18 may be fully or partially elasticized and/or may be foreshortened to create a void space between the topsheet 18 and the absorbent core 14. Exemplary structures including elasticized or foreshortened topsheets are described in more detail in U.S. Pat. No. 5,037,416 entitled "Disposable Absorbent Article Having Elastically Extensible Topsheet" issued to Allen et al. on Aug. 6, 1991; and U.S. Pat. No. 5,269,775 entitled "Trisection Topsheets for Disposable Absorbent Articles and Disposable Absorbent Articles Having Such Trisection Topsheets" issued to Freeland et al. on Dec. 14, 1993.

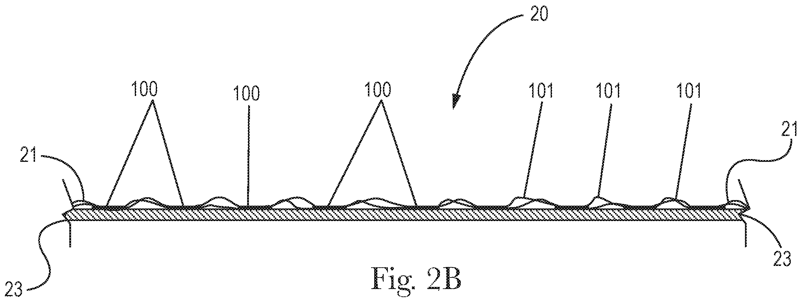

The backsheet 20 may be joined with the topsheet 18. The backsheet 20 may serve to prevent the exudates absorbed by the absorbent core 14 and contained within the diaper 10 from soiling other external articles that may contact the diaper 10, such as bed sheets and clothing. Referring to Fig. 2B, the backsheet 20 may be substantially impervious to liquids (e.g., urine) and may be formed of a laminate of a nonwoven 21 and a thin polymeric film 23 such as a thermoplastic film having a thickness of about 0.012 mm (0.5 mil) to about 0.051 mm (2.0 mils). Nonwoven 21 may be a nonwoven web as described herein. Suitable backsheet films include those manufactured by Tredegar Industries Inc. of Terre Haute, Ind. and sold under the trade names X15306, X10962, and X10964. Other suitable backsheet materials may include breathable materials that permit vapors to escape from the diaper 10 while still preventing liquid exudates from passing through the backsheet 20. Exemplary breathable materials may include materials such as woven webs, nonwoven webs, composite materials such as film-coated nonwoven webs, and microporous films such as manufactured by Mitsui Toatsu Co., of Japan under the designation ESPOIR and by EXXON Chemical Co., of Bay City, Texas, under the designation EXXAIRE. Suitable breathable composite materials comprising polymer blends are available from Clopay Corporation, Cincinnati, Ohio under the name HYTREL blend PI 8-3097. Other examples of such breathable composite materials are described in greater detail in PCT Application No. WO 95/16746, published on Jun. 22, 1995 in the name of E. I. DuPont. Other breathable backsheets including nonwoven webs and apertured formed films are described in U.S. Pat. No. 5,571,096 issued to Dobrin et al. on Nov. 5, 1996.

In some examples, the backsheet of the present invention may have a water vapor transmission rate (WVTR) of greater than about 2,000 g/24h/m2, greater than about 3,000 g/24h/m2, greater than about 5,000 g/24h/m2, greater than about 6,000 g/24h/m2, greater than about 7,000 g/24h/m2, greater than about 8,000 g/24h/m2, greater than about 9,000 g/24h/m2, greater than about 10,000 g/24h/m2, greater than about 11,000 g/24h/m2, greater than about 12,000 g/24h/m2, greater than about 15,000 g/24h/m2, measured according to WSP 70.5 (08) at 37.8 0C and 60% Relative Humidity.

Suitable nonwoven web materials useful in the present invention include, but are not limited to spunbond, meltblown, spunmelt, solvent-spun, electrospun, carded, film fibrillated, melt-film fibrillated, air-laid, dry-laid, wet-laid staple fibers, and other and other nonwoven web materials formed in part or in whole of polymer fibers, as known in the art. A suitable nonwoven web material may also be an SMS material, comprising a spunbonded, a melt-blown and a further spunbonded stratum or layer or any other combination of spunbonded and melt-blown layers, such as a SMMS or SSMMS etc. Examples include one or more layers of fibers with diameters below 1 micron (nanofibers and nanofiber layers); examples of these rise in combinations of SMS, SMNS, SSMNS or SMNMS nonwoven webs (where "N" designates a nanofiber layer). In some examples, permanently hydrophilic non-wovens, and in particular, nonwovens with durably hydrophilic coatings may be desirable. Typically, the suitable non-woven is air permeable. Typically the suitable nonwoven is water or liquid permeable, but may also be water impermeable by reason of fiber size and density, and hydrophobicity of the fibers. Water or liquid permeability may be enhanced by treatments to render the fibers hydrophilic, as discussed below.

The nonwoven web may be formed predominately of polymeric fibers. In some examples, suitable non-woven fiber materials may include, but are not limited to polymeric materials such as polyolefins, polyesters, polyamide, or specifically polypropylene (PP), polyethylene (PE), poly-lactic acid (PLA), polyethylene terephthalate (PET) and/or blends thereof. Nonwoven fibers may be formed of, or may include as additives or modifiers, components such as aliphatic polyesters, thermoplastic polysaccharides, or other biopolymers (bio-based or renewable polymers).

The individual fibers may be monocomponent or multicomponent. The multicomponent fibers may be bicomponent, such as in a core-and-sheath or side-by-side arrangement. Often, the individual components comprise aliphatic polyolefins such as polypropylene or polyethylene, or their copolymers, aliphatic polyesters, thermoplastic polysaccharides or other biopolymers.

Further useful nonwovens, fiber compositions, formations of fibers and nonwovens and related methods are described in U.S. Pat. No. 6,645,569 to Cramer et al., U.S. Pat. No. 6,863,933 to Cramer et al., U.S. Pat. No. 7,112,621 to Rohrbaugh et al..

Some polymers used for nonwoven fiber production may be inherently hydrophobic, and for certain applications they may be surface treated or coated with various agents to render them hydrophilic. A surface coating may include a surfactant coating. One such surfactant coating is available from Schill & Silacher GmbH, Boblingen, Germany, under the Tradename Silastol PHP 90.

Another way to produce nonwovens with durably hydrophilic coatings, is via applying a hydrophilic monomer and a radical polymerization initiator onto the nonwoven, and conducting a polymerization activated via UV light resulting in monomer chemically bound to the surface of the nonwoven as described in co-pending U.S. Patent Publication No. 2005/0159720.

Another way to produce hydrophilic nonwovens made predominantly from hydrophobic polymers such as polyolefins is to add hydrophilic additives into the melt prior to extrusion.

Another way to produce nonwovens with durably hydrophilic coatings is to coat the nonwoven with hydrophilic nanoparticles as described in co-pending applications U.S. Pat. No. 7,112,621 to Rohrbaugh et al. and in PCT Application Publication WO 02/064877.

Typically, nanoparticles have a largest dimension of below 750 nm. Nanoparticles with sizes ranging from 2 to 750 nm may be economically produced. An advantage of nanoparticles is that many of them can be easily dispersed in water solution to enable coating application onto the nonwoven, they typically form transparent coatings, and the coatings applied from water solutions arc typically sufficiently durable to exposure to water. Nanoparticles can be organic or inorganic, synthetic or natural. Inorganic nanoparticles generally exist as oxides, silicates, and/or carbonates. Typical examples of suitable nanoparticles are layered clay minerals (e.g., LAPONITE from Southern Clay Products, Inc. (USA), and Boehmite alumina (e.g., Disperal P2™ from North American Sasol. Inc.). According to one example, a suitable nanoparticle coated non-woven is that disclosed in the co-pending patent application Ser. No. 10/758,066 entitled "Disposable absorbent article comprising a durable hydrophilic core wrap" by Ponomarenko and Schmidt.

In some cases, the nonwoven web surface can be pre-treated with high energy treatment (corona, plasma) prior to application of nanoparticle coatings. High energy pre-treatment typically temporarily increases the surface energy of a low surface energy surface (such as PP) and thus enables better wetting of a nonwoven by the nanoparticle dispersion in water.

Notably, hydrophilic non-wovens are also useful in other parts of an absorbent article. For example, topsheets and absorbent core layers comprising permanently hydrophilic non-wovens as described above have been found to work well.

A nonwoven also may include other types of surface coating. In one example, the surface coating may include a fiber surface modifying agent that reduces surface friction and enhances tactile lubricity. Preferred fiber surface modifying agents are described in U.S. Pat. Nos. 6,632,385 and 6,803,103; and U.S. Pat. App. Pub. No. 2006/0057921.

According to one example, the nonwoven may comprise a material that provides good recovery when external pressure is applied and removed. Further, according to one example, the nonwoven may comprise a blend of different fibers selected, for example from the types of polymeric fibers described above. In some embodiments, at least a portion of the fibers may exhibit a spiral curl which has a helical shape. According to one example, the fibers may include bicomponent fibers, which arc individual fibers each comprising different materials, usually a first and a second polymeric material. It is believed that the use of side-by-side bi-component fibers is beneficial for imparting a spiral curl to the fibers.

In order to enhance softness perceptions of the absorbent article, nonwovens forming the backsheet may be hydroenhanced or hydroengorged. Hydroenhanced/hydroengorged nonwovens are described in U.S. Pats. Nos. 6,632,385 and 6,803,103, and U.S. Pat. App. Pub. No. 2006/0057921.

A nonwoven may also be treated by a "selfing" mechanism. By "selfing" nonwovens, high densities of loops (>150 in 2) may be formed which protrude from the surface of the nonwoven substrate. Since these loops act as small flexible brushes, they create an additional layer of springy loft, which may enhance softness. Nonwovens treated by a selfing mechanism are described in U.S. Pat. App. Pub. No. US 2004/0131820.

Any of the nonwoven types described herein may be used for the topsheet, backsheet outer layer, loops component in a hook-and-loop fastening system of an absorbent article.

The absorbent core generally may be disposed between the topsheet 18 and the backsheet 20. It may include one or more layers, such as a first absorbent layer 60 and a second absorbent layer 62.

The absorbent layers 60, 62 may include respective substrates 64, 72, an absorbent particulate polymer material 66, 74 disposed on substrates 64, 72, and a thermoplastic adhesive material 68, 76 disposed on and/or within the absorbent particulate polymer material 66, 74 and at least portions of the substrates 64, 72 as an adhesive for immobilizing the absorbent particulate polymer material 66, 74 on the substrates 64, 65.

The substrate 64 of the first absorbent layer 60 may be referred to as a dusting layer and has a first surface which faces the backsheet 20 and a second surface which faces the absorbent particulate polymer material 66. Likewise, the substrate 72 of the second absorbent layer 62 may be referred to as a core cover and has a first surface facing the topsheet 18 and a second surface facing the absorbent particulate polymer material 74.

The first and second substrates 64 and 72 may be adhered to one another with adhesive about the periphery to form an envelope about the absorbent particulate polymer materials 66 and 74 to hold the absorbent particulate polymer material 66 and 74 within the absorbent core 14.

The substrates 64, 72 may be of one or more nonwoven materials, and may be liquid permeable.

As illustrated in Fig. 2A, the absorbent particulate polymer material 66, 74 may be deposited on the respective substrates 64, 72 in clusters 90 of particles to form a grid pattern comprising land areas 94 and junction areas 96 between the land areas 94. Land areas 94 are areas where the thermoplastic adhesive material does not contact the nonwoven substrate or the auxiliary adhesive directly; junction areas 96 are areas where the thermoplastic adhesive material does contact the nonwoven substrate or the auxiliary adhesive directly. The junction areas 96 in the grid pattern contain little or no absorbent particulate polymer material 66 and 74. The land areas 94 and junction areas 96 can have a variety of shapes including, but not limited to, circular, oval, square, rectangular, triangular, and the like. First and second layers 60, 62 may be combined to form the absorbent core 14. Preferred absorbent articles and cores are described in U.S. Application Serial No. 12/141,122; U.S. Pat. Apps. Pub. Nos. 2004/0167486A1 and 2004/0162536; and PCT Pub. No. WO 2009/060384.

The foregoing description describes features of an absorbent article, any combination of which can be employed to enhance consumer perceptions of softness of the article. In addition, however, it is believed that manufacturing a nonwoven web, and using it as a component of an absorbent article including, *e.g.,* a topsheet 18 and/or backsheet 20 (*see* Figs. 2A, 2B), according to the following description, provides for enhancement of loft of the component, and has synergistic effects with respect to enhancing perceptions of softness of the article as a whole. At the same time, counterintuitively, features described below may enhance tensile strength of the nonwoven web, and consequently, of the topsheet, backsheet or other component formed of it. When attempting to improve softness signals, preserving or enhancing tensile strength of a nonwoven may be of particular interest in absorbent articles for at least two reasons. First, the nonwoven web may typically be required to sustain certain minimum tensile forces and undergo sufficiently low changes in dimension so as to be effectively processable in downstream manufacturing operations. Second, the nonwoven web typically may be a substantial contributor to structural integrity of a the manufactured product, such as a disposable diaper, in which the backsheet may be required to sustain forces resulting from application/donning on a wearer (*e.g.,* when a caregiver tugs on fastening members to apply a diaper), wearer movements, and weight and bulk contained and sustained by the backsheet when the diaper is loaded with the wearer's exudates.

As previously noted, referring to Fig. 2B, a backsheet 20 may be formed of a laminate of a nonwoven 21 and a thin polymeric film 23. The nonwoven and film may be bonded in the laminating process by adhesive or any other suitable means. In some examples, the polymeric film may have a thickness of about 0.012 mm (0.5 mil) to about 0.051 mm (2.0 mils). In order to achieve the desired overall visual appearance, the opacity and whiteness of the backsheet laminate may be enhanced by addition of, for example, calcium carbonate ($CaCO_3$) to the film during its formation. Inclusion of fine particles of $CaCO_3$ cause the formation of micropores about the particles upon stretching, or biaxial stretching in processing of the film, which serve to make the resulting film air- and vapor-permeable (thus, "breathable", reducing the likelihood of skin overhydration and thereby reducing the likelihood of conditions such as diaper rash). The $CaCO_3$ particles and the resulting micropores in the film also serve to enhance its opacity. Examples of suitable films include MICROPRO microporous films, and films designated BR137P and BR137U, available from Clopay Corporation, Mason, Ohio. In some examples, the polymeric film may be formed of components, and as described, in U.S. application Pub. No. 2008/0306463, and may include some or all of the features and/or components described therein, that reduce the film's vulnerability to glue "burn-through."

The nonwoven 21 may be formed from one or more resins of polyolefins, polyesters, polyamide including but not limited to polypropylene (PP), polyethylene (PE), and polyethylene terephthalate (PET), poly-lactic acid (PLA), and blends thereof. Resins including polypropylene may be particularly useful because of polypropylene's relatively low cost and surface friction properties of fibers formed from it (*i.e.,* they have a relatively smooth, slippery tactile feel). Resins including polyethylene may also be desirable because of polyethylene's relative softness/pliability and even more smooth/slippery surface friction properties. Relative each other, PP currently has a lower cost and fibers formed from it have a greater tensile strength, while PE currently has a greater cost and fibers formed from it have a lower tensile strength but greater pliability and a more smooth/slippery feel. Accordingly, it may be desirable to form nonwoven web fibers from a blend of PP and PE resins, finding a balance of the best proportions of the polymers to balance their advantages and disadvantages. In some examples, the fibers may be formed of PP/PE blends such as described in U.S. Pat. No. 5,266,392. Nonwoven fibers may be formed of, or may include as additives or modifiers, components such as aliphatic polyesters, thermoplastic polysaccharides, or other biopolymers.

The individual fibers may be monocomponent or multicomponent. The multicomponent fibers may be bicomponent, such as in a core-and-sheath or side-by-side arrangement. Often, the individual components comprise aliphatic polyolefins such as polypropylene or polyethylene, or their copolymers, aliphatic polyesters, thermoplastic polysaccharides or other biopolymers.

A batt may be formed from any of these resins by conventional methods, such as carding, meltblowing, spunlaying, airlaying, wet-laying etc. A preferred execution relates to spunbonding processes, in which the resin(s) are heated and forced under pressure through spinnerets. The spinnerets eject fibers of the polymer(s), which are then directed onto a moving belt; as they strike the moving belt they may be laid down in somewhat random orientations, but often with a machine-direction bias, to form a spunlaid batt. The batt then may be calender-bonded to form the nonwoven web.

Nonwovens formed of any basis weight may be used. However, as noted in the background, relatively higher basis weight, while having relatively greater apparent caliper and loft, also has relatively greater cost. On the other hand, relatively lower basis weight, while having relatively lower cost, adds to the difficulty of providing a backsheet that has and sustains a dramatic visual 3-dimensional appearance following compression in a package, and has suitable mechanical properties. It is believed that the combination of features described herein strikes a good balance between controlling material costs while providing a dramatic visual 3-dimensional appearance and suitable mechanical properties.

It is believed that the features of consolidating bond shapes and patterns described herein may be particularly useful in applications of nonwovens of relatively low basis weights in some applications, in that it is believed that such features provide a way to enhance loft while reducing, or at least without adding, basis weight. Accordingly, for such applications, a nonwoven having a basis weight from 6.0 to 50 gsm, more preferably from 8.0 to 35 gsm, even more preferably from 9.0 to 25 gsm, and still more preferably from 10 to 20 gsm may be used. When used as a component of an absorbent article such as a topsheet, a lower basis weight nonwoven may provide strikethrough superior to that of a higher basis weight nonwoven. A lower basis weight nonwoven may be preferable to a higher basis weight one when used, for example, as a component of a zero-strain stretch laminate, because it will be more accommodating of an activation/incremental stretching process. In other applications, such as, for example, use of nonwovens to form products such as disposable clothing articles, wipes or dusters, higher basis weights up to 100 gsm, or even 150 gsm, may be desired. It is believed that the features of bonding protrusions, bonding shapes and bonding patterns described herein may have beneficial effects on loft and/or softness perception, even with nonwovens of such higher basis weights. Optimal basis weight is dictated by the differing needs in each application, and cost concerns.

It is believed that the desired overall visual softness signals of a backsheet laminate may be better achieved when the backsheet laminate is substantially white in color, and has an Opacity of at least 45%, more preferably at least 70%, even more preferably at least 73%, and still more preferably at least 75%, as measured by the Opacity Measurement Method set forth below. Accordingly, it may be desirable to add a white-tinting/opacifying agent also to the polymer(s) forming the polymeric film, and to the polymer(s) supplying the spinnerets used to form the fibers of the nonwoven web.

It may be desirable that a white-tinting/opacifying agent be added to the polymer resin that is spun to make the nonwoven. Adjusting the opacity of the nonwoven web, through addition of an opacifying agent, may be desirable, such that the nonwoven web has an Opacity of at least 10%, more preferably at least 18%, and still more preferably at least 40%.

While a variety of whitening/opacifying agents may suffice, it is believed that titanium dioxide ($TiO_2$) may be particularly effective because of its brightness and relatively high refractive index. It is believed that addition of $TiO_2$ to the polymer(s) from which the fibers are to be formed, in an amount up to 5.0% by weight of the nonwoven, may be effective to achieve the desired results. However, because $TiO_2$ is a relatively hard, abrasive material, inclusion of $TiO_2$ in amounts greater than 5.0% by weight may have deleterious effects, including wear and/or clogging of spinnerets; interruption and weakening of the structure of the fibers and/or calender bonds therebetween; undesirably increasing the surface friction properties of the fibers (resulting in a less smooth tactile feel); and unacceptably rapid wear of downstream processing equipment components. It is believed that the increased opacity provided by whitener helps to produce a visually distinctive, soft appearance of the nonwoven. It also may be desired in some applications that a coloring or tinting agent be added to one or more the polymer resin(s) from which the nonwoven fibers will be spun.

Opacity can also be enhanced by using fiber having cross-sectional shapes other than round and solid (non-hollow) geometries, namely trilobal or multilobal cross-sections, or hollow configurations or combinations thereof. Those non-circular cross-sectional shapes can also provide advantages in terms of loft and compression resilience.

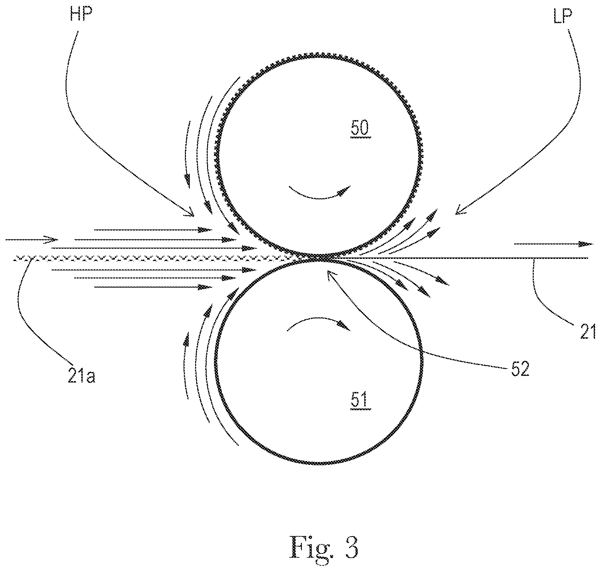

Spunbonding includes the step of calender-bonding the batt of spunlaid fibers, to consolidate them and bond them together to some extent to create the web as a fabric-like structure and enhance mechanical properties *e.g.*, tensile strength, which may be desirable so the material can sufficiently maintain structural integrity and dimensional stability in subsequent manufacturing processes, and in the final product in use. Referring to Fig. 3, calender-bonding may be accomplished by passing the batt 21a through the nip between a pair of rotating calender rollers 50, 51, thereby compressing and consolidating the fibers to form a nonwoven web 21. One or both of the rollers may be heated, so as to promote heating, plastic deformation, intermeshing and/or thermal bonding/fusion between superimposed fibers compressed at the nip. The rollers may form operable components of a bonding mechanism in which they are urged together by a controllable amount of force, so as to exert the desired compressing force/pressure at the nip. In some processes an ultrasonic energy source may be included in the bonding mechanism so as to transmit ultrasonic vibration to the fibers, again, to generate heat energy within them and enhance bonding.

One or both of the rollers may have their circumferential surfaces machined, etched, engraved or otherwise formed to have thereon a bonding pattern of bonding protrusions and recessed areas, so that bonding pressure exerted on the batt at the nip is concentrated at the bonding surfaces of the bonding protrusions, and is reduced or substantially eliminated at the recessed areas. The bonding surfaces have bonding surface shapes. As a result, an impressed pattern of bonds between fibers forming the web, having bond impressions and bond shapes corresponding to the pattern and bonding surface shapes of the bonding protrusions on the roller, is formed on the nonwoven web. One roller such as roller 51 may have a smooth, unpatterned cylindrical surface so as to constitute an anvil roller, and the other roller 50 may be formed with a pattern as described, to constitute a bonding pattern roller; this combination of rollers will impart a pattern on the web reflecting the pattern on the bonding pattern roller. In some examples both rollers may be formed with patterns, and in particular examples, differing patterns that work in combination to impress a combination pattern on the web such as described in, for example, U.S. Pat. No. 5,370,764.

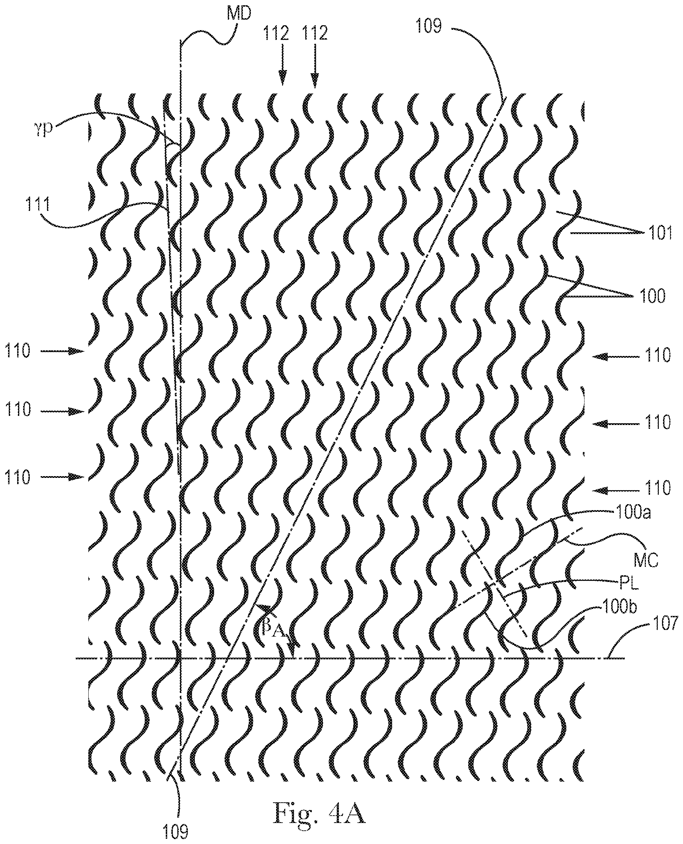

A repeating pattern of bonding protrusions and recessed areas such as, for example, depicted in Fig. 4A, may be formed onto a bonding roller 50 (Fig. 3). The pattern depicted in Fig. 4A is one example of the patterns. The "S"-shaped bonding shapes 100 depicted in Fig. 4A depict raised surfaces of bonding protrusions on a roller, while the areas between them represent recessed areas 101. The bonding shapes 100 of the bonding protrusions impress like-shaped bond impressions on the web in the calendering process.

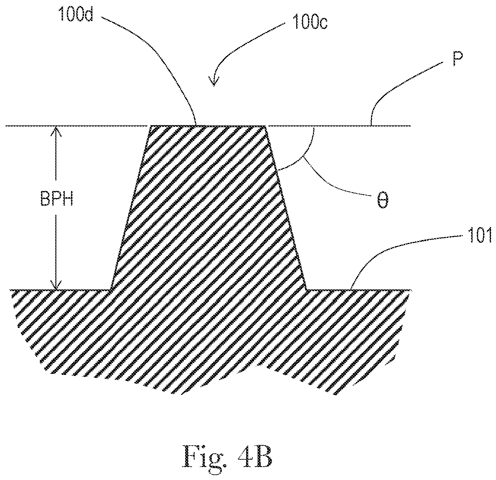

Referring to Fig. 4B, the bonding protrusions 100c on a roller will have a height BPH, which may be expressed as a difference between the radius of the roller at the outermost (bonding) surfaces 100d of the bonding protrusions 100c, and the radius of the roller at the recessed areas 101. The height may be adjusted with the objective of minimizing the amount of material that must be removed from the roller surface by machining or etching to create the desired shapes and pattern, while still providing for sufficient clearance between the roller bearing the bonding protrusions and the opposing roller, at the recessed areas 101, to accommodate passage of the batt through the nip in areas of the batt not to be bonded (i.e., at the recessed areas), without substantially compressing it, and for purposes herein, providing air passageways between the bonding protrusions in the nip. For webs of the type and basis weight contemplated herein, a bonding protrusion height BPH between 0.3 mm and 1.0 mm may be desired, or more preferably, a bonding protrusion height between 0.5 mm and 0.9 mm, or even a bonding protrusion height between 0.6 mm and 0.8 mm. The bonding surfaces of the bonding protrusions may have an average area between 0.3 mm$^2$ and 10 mm$^2$. The sides of the bonding protrusions may be formed with an angled slope (supporting slope) when viewed in cross section through the height thereof, which may enhance the strength of the protrusions and improve the longevity of the roller. The angle (supporting slope angle θ) may be from 55 degrees to 80 degrees, and is the smallest identifiable angle between a plane P that is parallel to the roller's axis of rotation and perpendicular to its radius, and the shortest line segment lying along the slope of the protrusion and connecting the bonding surface 100d and a recessed area 101, as illustrated in Fig. 4B. A bonding protrusion having a supporting slope angle larger than 80 degrees may adversely affect bond formation, and may not have the desired level of advantage in bond protrusion strength/longevity, while a supporting slope angle less than 55 degrees may also adversely affect bond formation and will undesirably result in reduction of the effective size of the air passageway between bonding protrusions through the nip.

Nonwoven webs of the type contemplated herein may be calender-bonded at line speed greater than 300 m/min., or 600 m/min., or even 800 m/min., or more, depending upon nonwoven web composition, basis weight, bonding pattern, and equipment and process variables selected. Referring again to Fig. 3, it will be appreciated that at such speeds, the batt 21a and the surfaces of rollers 50, 51 will entrain surrounding air and move it toward the nip 52, as suggested by the arrows. Features of a bonding roller 50, such as bonding protrusions as described herein, will also entrain air as the roller rotates, adding to this effect. It is believed that, as entrained air is carried toward the nip by the batt and the bonding roller, the momentum of the entrained air and the decreasing space between the rollers as the nip is approached creates a first zone HP of relatively higher, and increasing, air pressure in front of the nip 52. A portion of the entrained air under such higher pressure will be urged into and further compressed in the nip 52, within the recessed areas 101 of the bonding pattern on the roller, and within the interstices between the fibers passing through the nip. It is believed that, as nonwoven web 21 exits the nip 52, the compressed air exiting the nip encounters a second zone LP of relatively lower pressure on the exit side, and accelerates away from the nip in all unobstructed directions as a result. Thus, it is believed that substantial air entrainment, air compression and complex air flows of relatively high velocity occur within and about the batt 21a and web 21 as a result of movement of the batt and rotation of the calender rollers in the calender-bonding process.

It is believed that features of a bonding roller including the bonding protrusions affect these air flows. In the nip, the profiles of bonding protrusions present obstructions to airflow, while the recessed areas between the bonding protrusions present passageways. Thus, it is believed that for certain configurations, shapes, and positions of bonding protrusions, as will be reflected in the bond impressions created in the web, rotational orientation(s) and repeating patterns of the bonding shapes can be selected and formed to have a beneficial effect on these air flows. It is believed, further, that patterns of bonding protrusions having bonding surface shapes with certain features, reflected in the bonding surfaces and the cross sections of the protrusions along planes substantially parallel with the bonding surfaces, rotational orientations relative the plane approximated by the web surface, and spacing, may be employed to channel these air flows in a way that causes them to reposition the fibers during the calender bonding process, such as by teasing or fluffing the fibers, thus providing an enhanced calender-bonded nonwoven web having greater loft/caliper than a similar nonwoven web having other consolidated bond shapes and patterns, all other variables being the same.

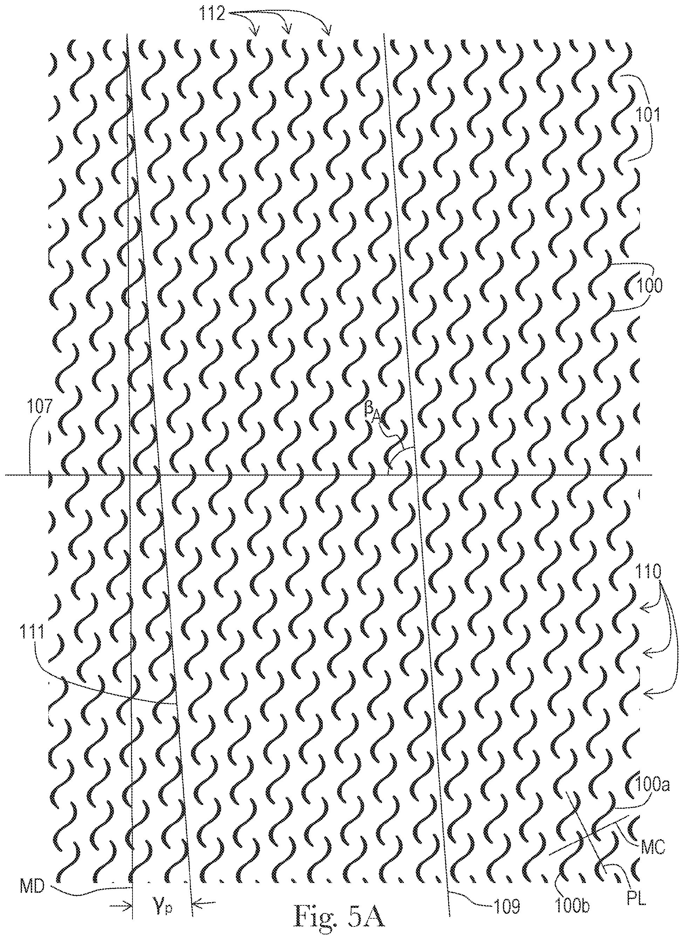

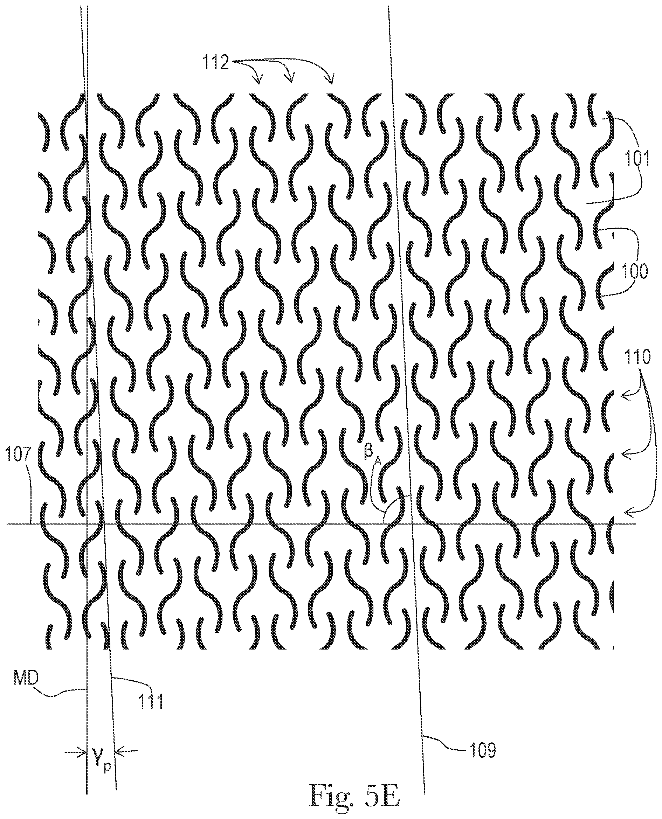

Figs. 5A-5D depict another non-limiting example of a bonding pattern and bonding shapes that will be reflected in bond shapes of bond impressions in a nonwoven web. Fig. 5E depicts another non-limiting example. Bonding shapes 100 represent the shapes of bonding surfaces of bonding protrusions that may be imparted to a bonding roller by etching, machining or other methods. Such bonding protrusions on a bonding roller will impress bond impressions into a web, of like bond shapes, arranged in a like bonding pattern. Without intending to be bound by theory, it is believed that certain aspects and features of the shapes and pattern may further enhance the beneficial effect described above.

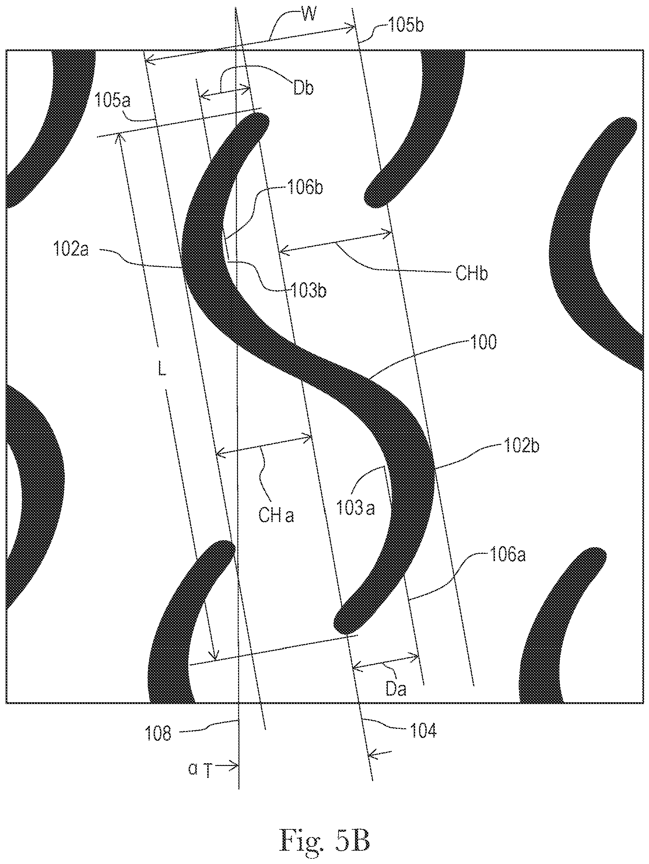

Referring to Fig. 5B, the bonding shape 100 has a greatest measurable length L, which is measured by identifying a shape length line 104 intersecting the perimeter of the shape at the two points of intersection that are the greatest distance apart that may be identified on the perimeter, i.e., the distance between the two farthest-most points on the perimeter. The bonding shape 100 has a greatest measurable width W which is measured by identifying respective shape width lines 105a, 105b which are parallel to shape length line 104 and tangent to the shape perimeter at one or more outermost points that are most distant from shape length line 104 on either side of it, as reflected in Fig. 5b. It will be appreciated that, for some shapes (*e.g.,* a semicircle), one of shape width lines 105a, 105b may be coincident/colinear with shape length line 104. Greatest measurable width W is the distance between shape width lines 105a, 105b. Shapes within the scope of the present invention have an aspect ratio of greatest measurable length L to greatest measurable width W of at least 2.0 in order to have characteristics of an airfoil and not merely an obstruction to air flow. At the same time, it may be desired that the aspect ratio have an upper limit so that the bonding protrusions create multiple branched air passageways through the nip, promoting maximum airflow and turbulence at the same time. Thus, it may be desired that the aspect ratio be from 2.0 to 3.0, more preferably from 2.2 to 2.8, and even more preferably from 2.3 to 2.7. The bond shapes and sizes impressed on the nonwoven web will reflect and correspond with the bonding shapes 100 and sizes thereof on the roller.

Still referring to Fig. 5B, a bonding shape 100 may have a shape perimeter with a convex portion 102a and/or 102b, lying on at least one side of the shape length line 104. Fig. 5B reflects also that the convex portion may have a varying radius or radii. The varying radius/radii of the convex portion 102a and/or 102b may impart the shape perimeter with similarities to the profile of the camber of an airfoil in cross section. Viewed another way, the cross-sectional profile of an airfoil has a convex portion and is asymmetric about any line or axis that traverses the profile, which can be identified. The convex portion 102a and/or 102b may have a camber height CHa and/or CHb measured as the distance between shape length line 104 and the shape width line 105a and/or 105b that is tangent to the convex portion 102a and/or 102b. It is believed that, for maximum beneficial impact on airflow, it may be desirable that the ratio between a camber height CH and greatest measurable length L be 0.45 or less, more preferably 0.40 or less, even more preferably 0.35 or less, but greater than zero. It is believed that a bonding protrusion having a cross section along a plane parallel the bonding surface, fitting this description, arranged in a repeating pattern, has beneficial effects on acceleration and deceleration of air through nonwoven fibers at and about the nip. Again, the bond shapes and sizes impressed on the nonwoven web will reflect and correspond with the bonding shapes and sizes on the roller.

In one example (not shown), the shape perimeter may have a convex portion, with or without a varying radius, on both sides of shape length line 104, such that it resembles the contour of an airfoil with symmetrical camber, in cross section. In another example (not shown), the shape perimeter may have a convex portion on one side of shape length line 104 and a straight portion on or on the other side of shape length line 104, such that it resembles the contour of an airfoil/aircraft wing with asymmetrical camber, in cross section. In another example, the shape perimeter may have a convex portion 102a and/or 102b on one side of shape length line 104 and a concave portion 103a and/or 103b disposed substantially opposite the concave portion, as reflected in Fig. 5B, such that it resembles the contour of an airfoil/aircraft wing with asymmetrical camber and relatively high-loft, low-speed features, in cross section.

The extent of the concavity of concave portion 103a and/or 103b may be quantified by measuring the depth thereof, relative the greatest measurable length. The concavity depth Da and/or Db may be measured by identifying a shape concavity line 106a and/or 106b that is parallel with the shape length line 104 and tangent to the deepest point along the concave portion 103a and/or 103b. The concavity depth Da and/or Db is the distance between the shape length line and the shape concavity line 106a and/or 106b. The extent of the concavity of concave portion 103a and/or 103b may be expressed as a ratio of concavity depth Da and/or Db to shape length L (hereinafter, "concavity depth ratio"). Although shapes that do not have a concave portion 103a, 103b are contemplated, it may be desirable that a bonding shape has a concave portion having a concavity depth ratio between 0.00 and 0.30, more preferably between 0.00 and 0.25, and even more preferably between 0.00 and 0.20. Again, the bond shapes and sizes impressed on the nonwoven web will reflect and correspond with the bonding shapes and sizes on the roller.

While the explanation above refers to bonding protrusions and resulting consolidated bond shapes in the web, which have bonding shape/bond shape perimeters following "convex" and/or "concave," curvature, suggesting smooth curves, it may be appreciated that the effect may be substantially realized by approximating smooth curves with chains of straight line segments. Accordingly, each of the terms "convex" and "concave" herein includes a portion of a shape perimeter formed of a chain of 3 or more straight line segments lying on one side of a shape length line and connected end-to-end, that is each a chord of a smooth convex or concave curve lying on one side of the shape length line, or portion of a curve lying on one side of the shape length line that does not include an inflection point.

Without intending to be bound by theory, it is believed that calender roller bonding protrusions having bonding shapes with one or more features as described above have aerodynamic effects on air flow in and about the nip, that cause acceleration and deceleration of air in and about the interstices of the nonwoven fibers in a way that repositions the fibers, and may effect teasing or fluffing, adding loft and caliper.

Additionally, the rotational orientations of the protrusions affect the orientations of the bonding protrusions at the nip, and it is believed that this has an impact. Bonding shapes 100 and the bonding protrusions supporting them may be arranged along an individual shape tilt angle relative the machine and cross directions. Without intending to be bound by theory, it is believed that the shape tilt angle should not exceed a certain amount for the bonding protrusion to have maximum beneficial effect on air flow. Referring again to Fig. 5B, the shape tilt angle $\alpha_T$ may be expressed as the smaller angle formed by the intersection of an axis 108 along the machine direction and the shape length line 104. It is believed, that the shape and the shape tilt angle have cooperating effects on the air flow. In the case of an asymmetric bonding shape, such as the described airfoil-like shape, it is believed that this asymmetric bonding shape is sufficient for effecting the desired changes in air flow. However, a rotational orientation with a tilt angle of more than zero may enhance the effect. With respect to a bonding shape that is not asymmetric, it is believed that the shape tilt angle $\alpha_T$ provides the desired effects on air flow, such that it then should not be less than 1 degree and should not exceed 40 degrees, more preferably not exceed 30 degrees, and still more preferably not exceed 20 degrees. It is believed that a shape tilt angle within this range effectively provides air flow through the nip, while at the same time, imparts cross-direction vector components to air flows through the nip. Conversely, a shape tilt angle greater than 40 degrees (combined with the elongate aspect ratio of the shape as described above) may create too much of an obstruction to air flow through the nip to have a beneficial effect, and even greater shape tilt angles combined with sufficient density of bonding protrusions may have the effect of creating enough obstruction at the nip to substantially divert airflow from the nip, *i.e.,* toward the sides of the bonding rollers, rather than through the nip. The bond shapes and rotational orientations impressed on the nonwoven web will reflect and correspond with the bonding shapes and rotational orientations on the roller.

It is believed that air flows having cross-direction vector components flowing across or through the batt/web as it passes through and exits the nip may urge fibers in the cross-direction, helping add loft, caliper and/or cross direction tensile strength. It will be appreciated that the fibers of many nonwoven batts are laid down in the nonwoven web manufacturing process with a general machine direction bias, which tends to result in the finished web having relatively greater machine direction tensile strength, and relatively less cross direction tensile strength. Thus, any process that tends to impart some added cross-direction orientation to the fibers prior to or during bonding may be useful for increasing cross direction tensile strength, bringing about better balance between machine direction tensile strength and cross-direction tensile strength, and adding loft such as by repositioning of the fibers in the z-direction. It is believed that, for best results, it may be even more desirable that shape tilt angle $\alpha_T$ is between 5 degrees and 15 degrees, more preferably between 8 degrees and 12 degrees, and even more preferably between 9 degrees and 11 degrees, for the most beneficial effects on airflow at the line speeds contemplated herein. The rotational orientation of the bonding pattern impressed on the nonwoven web will reflect and correspond with the rotational orientation of the bonding pattern on the roller.

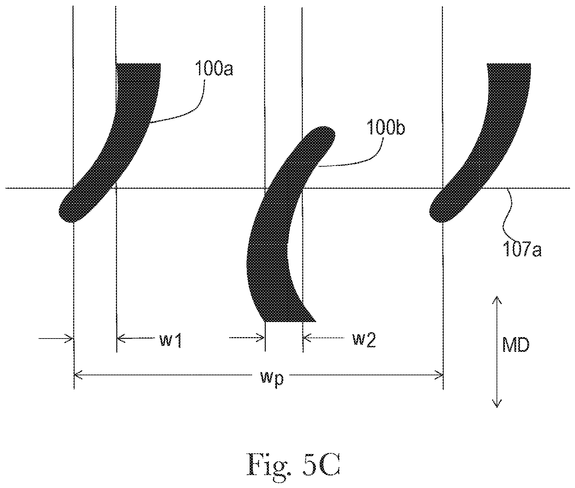

As suggested above, in order to gain the benefit of energy from a substantial mass of air under pressure flowing through the nip, it is also believed desirable that a pattern of bonding protrusions not be excessively obstructive of air flow through the nip, nor that it remove too much energy from the air flow by overly slowing, or halting, and absorbing the energy from, forward (machine-direction) momentum of air flows. Referring to Fig. 5C, a nip line 107a along the cross direction is identified along a pattern where the bonding shapes occupy the greatest proportion of distance along a cross direction line that can be identified in a pattern. Thus, nip line 107a located as shown represents a cross-direction line along which bonding protrusions presented the greatest amount of obstruction that can be identified in a particular pattern, to air flow through the nip, during the bonding process. A repeating series of shapes can be identified; in this example, the repeating series consists of the two shapes 100a and 100b. Widths $w_1$ and $w_2$ of the identified shapes 100a and 100b in the repeating series reflect restriction of air flow along the nip line 107a. Width $w_p$ is the width of the entire repeating series, including the distances between the bonding shapes. The proportion of maximum restriction along the nip length for the pattern is reflected by the ratio $(w_1 + w_2 ... + w_n)/ w_p$, referred to herein as the nip airflow restriction ratio (where each "$w_n$" is the cross-direction width along the nip line 107a one of the bonding shape perimeters along the nip line that constitutes the repeating series. In order that a bonding pattern allows for effective air flow through the nip in order to take advantage of energy of moving air, it may be desirable that the nip airflow restriction ratio be 0.40 or less, more preferably 0.30 or less, and even more preferably 0.25 or less. The bond shapes, rotational orientations and density/numerosity per unit surface area of bond impressions on the nonwoven web will reflect and correspond with the bonding shapes, rotational orientations and density/numerosity per unit surface area of bonding protrusions on the roller, and thus, also reflect the airflow restriction ratio.

It is also believed that arranging the bonding protrusions in a pattern such that a straight, unobstructed passageway between them exists along recessed areas 101 at the nip, at least partially along the machine direction, may have beneficial effects. Referring again to Fig. 5A and 5E, it can be seen that each example has at least one cross-nip airflow line 109 that can be identified, that intersects no bonding shape, and intersects a cross direction axis 107 at an angle such that it has a machine direction vector component. In each depicted example cross-nip airflow line 109 intersects cross direction axis 107 to form a smaller angle, identified herein as cross-nip airflow angle $\beta_A$. It is believed that cross-nip airflow angle $\beta_A$ should be preferably greater than 45 degrees, more preferably between 50 degrees and 90 degrees, and even more preferably between 60 degrees and 90 degrees. It is believed desirable that cross-nip airflow line 109 should extend indefinitely without intersecting a bonding shape 100, but at a minimum, past at least 8 rows 110 of bonding shapes 100 without intersecting a bond shape. The cross-nip airflow line 109 as described above reflects an uninterrupted air passageway through the nip. Again, geometric features of the bond shapes and pattern on the nonwoven web will reflect and correspond with those of the shape, size, rotational orientation, density and arrangement of the bond shapes 100. Another aspect of the bonding shapes and pattern depicted in, *e.g.,* Fig. 5Ais that they may have any combination of the above-described aspect ratios, maximum nip airflow restriction ratio (0.40 or less), shape asymmetry, shape tilt angles, and other features, and may also reflect use of adjacent pairs of bonding protrusions that define air passageways through the nip that alternately narrow and widen, or converge and diverge, in the manner of a venturi. For example, referring again to Fig. 5A, two adjacent bond shapes 100a, 100b may be identified. Herein, "adjacent" means that at least portions of the perimeters of a pair of shapes face each other with no intervening shapes between them; and that the pair of shapes has machine-direction overlap. The pair of shapes has machine-direction overlap if one or more cross-direction lines 107 that are tangent to and/or cross the perimeters of each of the shapes may be identified. A minimum passageway clearance line MC may be identified connecting the perimeters of the shapes 100a, 100b, at the location where the shortest measurable distance between the perimeters exists. The minimum passageway clearance line MC will necessarily meet the perimeter of each of the adjacent shapes where line MC is normal to the perimeter, and line MC identifies the point of greatest constriction of an air passageway between the shapes (*i.e.,* through the corresponding bonding protrusions) proximate and through the nip. A passageway line PL may be identified, perpendicular to the minimum passageway clearance line MC and lying between the adjacent shapes 100a, 100b.

The minimum passageway clearance line MC crosses and identifies a "venturi passageway" if the perimeter of each of the adjacent shapes 100a, 100b diverges away from the passageway line PL moving along the perimeter away from the minimum clearance line MC in both directions. It can be seen in Fig. 5A that adjacent shapes 100a, 100b embody this feature.

Without intending to be bound by theory, it is believed that such venturi passageways have the effect of causing localized zones of acceleration and deceleration, and increases and decreases in pressure, as well as turbulence, of air as it passes through the nip. It is believed that these effects serve to tease and/or fluff the fibers of the batt and web about the nip.

For purposes of downstream handling and manufacturing processes using the nonwoven web product, it may be desirable to ensure that no line along the machine direction exists along the nonwoven web surface that is indefinitely long without intersecting a bond impression. This condition (indefinitely long machine direction strip of web without bonds) may result in relatively long lengths of unbonded fibers that may be prone to moving away from a cutting knife in downstream machine direction web slitting operations, resulting in a poorly defined or sloppy slit edge. Additionally, such long, unbonded fibers may also separate from a manufactured edge or slit edge of the web (fraying), which may cause other difficulties in downstream operations. To avoid this condition, it may be desirable to impart a pattern tilt angle $\gamma_P$ to the bonding pattern. Referring to Fig. 5A, pattern tilt angle $\gamma_P$ may be expressed as the smaller angle formed by the intersection of a line 111 tangent to repeating, similarly oriented shapes in columns 112, and a machine direction axis MD. To avoid the above-mentioned problems, it may be desirable that pattern tilt angle $\gamma_P$ be greater than 0 degrees. The effect may be seen in Fig. 5A, where line MD alternately crosses unbonded pathways and columns 112 of bond shapes 100. A pattern tilt angle greater than 0 degrees will ensure that an indefinitely long machine direction strip of web without bonds will not exist. To avoid creating complications with respect to the air flow benefits of the pattern, however, it may be desirable to limit pattern tilt angle $\gamma_P$ to 6 degrees or less, more preferably 5 degrees or less, and even more preferably 4.5 degrees or less. Again, features of the bond pattern on the nonwoven web including pattern tilt angle will reflect and correspond with those of the pattern and pattern tilt angle $\gamma_P$ on the roller.

It has been discovered, also, that imparting a pattern tilt angle $\gamma_P$ to the pattern as described above has the effect at smoothing out variations of bonding pressure between the bonding surfaces and the anvil roller, along the nip (cross direction) as the calender roller and the anvil roller rotate. This effect is analogous to the force- and torque-smoothing effect of parallel axis helical meshing gears as compared with parallel axis spur meshing gears. The benefit for purposes of calender bonding a nonwoven is more evenly distributed bonding pressure, yielding consistency of bonding and consistency of formation of bond shapes impressed into the nonwoven, and even wear and longevity of the calender bonding roller.

Another functional feature of the pattern examples depicted in Figs. 5A and 5E is the machine-direction overlap between rows. It was previously believed that increasing machine direction overlap would undesirably compromise nonwoven loft by creating larger areas in which bond-to-bond distance is substantially reduced (*i.e.,* in the areas of machine-direction overlap), resulting in more frequently and tightly-bound fibers. Surprisingly, however, it has been discovered that imparting a machine direction overlap between the rows of the pattern of from 15 percent to 25 percent, and using the roller bearing the pattern to calender bond a nonwoven, desirably maintains the loft-creating advantages of the other pattern features described herein, while substantially improving tensile strength and reducing neckdown.

The features described above apply to the shapes of bonding surfaces of bonding protrusions in a pattern on a bonding roller, and it will be understood that these features are impressed by the roller into the nonwoven batt to form bond impressions having bond shapes and bonds threat, to form the calender-bonded nonwoven web. As impressed into a nonwoven web, the bonding shapes are reflected as bond shapes, and are identifiable, and measurable in the web, in laminates that include such nonwoven web as a composite layer, and in composite products made from such nonwoven web and/or such laminates.

An additional aspect that it believed important is bonding area of a roller, reflected in bond area on the web. Imagining a pattern of bonding surfaces having shapes reflected in, for example, Figs. 5A and 5E impressed on a surface of a nonwoven web, bonding area and bond area is the area occupied by the bonding shapes on the roller and bond shapes impressed on the surface of the web. In the field of nonwoven web manufacturing, bonding area is often expressed as a percentage, calculated as:

$$Bonding\ Area\ \% = \left[ \frac{(bonding\ area\ within\ a\ surface\ area\ unit)}{(total\ surface\ area\ of\ the\ surface\ area\ unit)} \right] \times 100\%$$

The bonding area reflects the combination of bonding protrusion density (number of bonding protrusions per unit surface area) and average surface area of the bonding shapes 100 in the unit surface area. Thus, increasing the number of bonding protrusions and/or increasing the surface area of the individual bond shapes 100 increases the bonding area, and *vice versa.* It is believed that bonding area has an impact on the entrainment of air as well as the proportion of entrained air carried toward the nip, which will pass through the nip. If bonding area is relatively greater, this means that more and/or larger bonding protrusions are present at the nip point at any time to obstruct air flow through the nip; conversely, if bonding area is relatively less, this means that fewer and/or smaller bonding protrusions are present at the nip point at any time to obstruct air flow through the nip. Bond area has another effect as well. Increasing bond area increases the number and proportion of the fibers in the nonwoven web that are bonded together, and *vice versa.* Within a certain range of bond area, tensile strength of the nonwoven web in the machine and/or cross directions may be increased by increasing the bond area. However, bending stiffness of the nonwoven web may be correspondingly increased, and loft decreased - compromising the soft feel and/or appearance of the nonwoven. In order to best realize the benefits of air flow, air compression and channeling believed to be occurring through use of the bond shapes described herein, enhancing loft, while still imparting satisfactory tensile properties to the web, it is believed that bonding area should be in the range of 8.0% and 20%, more preferably between 10 % and 18%, and even more preferably between about 12% and 16%. At the line speeds contemplated herein, and relative to the bonding area, the average surface area per bonding shape affects bonding area and bonding protrusion density. It is believed desirable that the average bonding shape 100 surface area be in the range of from 2.5 mm$^2$ to 8.0 mm$^2$, more preferably from 3.0 mm$^2$ to 6.0 mm$^2$, and even more preferably from 3.5 mm$^2$ to 5.5 mm$^2$. Correspondingly, it is believed desirable that the density of the bonding protrusions, and correspondingly, the impressed bond shapes (bond number density), be from 1.5 bonding protrusions/cm$^2$ to 6.0 bonding protrusions/cm$^2$, more preferably from 2.0 bonding protrusions/cm$^2$ to 5.0 bonding protrusions/cm$^2$, and even more preferably from 2.5 bonding protrusions/cm$^2$ to 4.0 bonding protrusions/cm$^2$.

In one particular non-limiting example, the bonding pattern on a calender roller may have the appearance of Figs. 5A-5D, and have the following combination of features:

bonding area approximately 13.9%
bonding protrusion height BPH approximately 1 mm
shape aspect ratio approximately 2.5
shape tilt angle $\alpha_T$ approximately 10 degrees
nip airflow restriction ratio approximately 0.19
cross-nip airflow angle $\beta_A$ approximately 85.7 degrees
pattern tilt angle $\gamma_P$ approximately 4.3 degrees
machine direction overlap approximately 19 percent

Experiments with a calender bonding roller having an engraved pattern with the features described immediately above showed that it imparted improved (reduced) neckdown and improved tensile strength with substantially the same loft/caliper, to two spunlaid nonwoven webs with basis weights of 13 gsm and 25 gsm, as compared with a calendar bonding roller engraved with a pattern as depicted Fig. 4A. The lighter web comprised all monocomponent polypropylene spunlaid filaments. The heavier web comprised a layered combination of monocomponent polypropylene and bicomponent polypropylene/polypropylene filaments. The improvements in reduced neckdown and improved tensile strength were surprising and dramatic. Machine direction tensile strength increased by 15% and 6%, for the lighter and heavier webs, respectively. Cross direction tensile strength increased by 18% and 11%, for the lighter and heavier webs, respectively. Neckdown improved *(i.e., reduced)* by 16% and 40%, for the lighter and heavier webs, respectively.

It is also believed that the speed of travel of the batt toward the bonding nip (batt line speed) is important. It will be appreciated that, if the batt line speed is too slow, air mass entrained by the batt as it approaches the nip will not have sufficient momentum to maintain a large enough zone of sufficiently elevated air pressure at the entry side effective to ensure that substantial air mass is urged through the nip, rather than being merely urged around the nip and the rollers along alternate pathways. Accordingly, it is believed that line speed at which the batt is conveyed toward the nip should be equal to or greater than 300 meters/minute, more preferably, equal to or greater than 600 meters/minute, and even more preferably, equal to or greater than 800 meters/minute.

It is believed that use of a calender roller having bonding patterns and bonding shapes as described herein take advantage of air flows resulting from entrainment of air along a moving nonwoven batt and calender rollers, and air compression, that occur during calender-bonding, in a way that causes the resulting nonwoven web to have enhanced loft and a soft feel. It is believed also that the bonding shapes need not be all of like kind or rotational orientation, but rather, that suitable combinations of differing shapes including bonding shapes having features as described herein, and optionally, in combination with other shapes, may be used and included. Employment of the described features may reduce or eliminate a need for other loft enhancement processes, such as hydroengorgement or hydroentanglement - which may save costs of additional equipment and operation.

Test/Measurement methods

Basis Weight

The "basis weight" of a nonwoven web is measured according to the European standard test EN ISO 9073-1:1989 (conforms to WSP 130.1). There are 10 nonwoven web layers used for measurement, sample size 10x10 $cm^2$.

Thickness

The "thickness" of a nonwoven web is measured according to the European standard test EN ISO 9073-2:1996 (conforms to WSP 120.6) with following modification: the overall weight of upper arm of the machine including added weight is 130 g.

MD/CD Ratio

The "MD/CD ratio" is the ratio of material's tensile strength at peak in the MD and CD direction. Both were measured according to the EDANA standard method WSP 110.4-2005, where sample width is 50 mm, jaw distance is 100 mm, speed 100 mm/min and preload 0,1N. MD/CD ratio = tensile strength at peak in MD[N/5cm] / tensile strength at peak in CD[N/5cm]

Softness

The "softness" of a nonwoven web may be measured using to the "Handle-O-Meter" test. The test used herein is the INDA 1ST 90.3-01. The lower the value, the softer is the web.

Volume Mass

The "volume mass" is the ratio of basis weight and thickness and indicates the bulkiness and fluffiness of the product, which are important qualities of the nonwoven web according to the invention. The lower the value, the bulkier is the web.

$$\text{Volume mass } [kg/m^3] = \text{basis weight } [g/m^2] \text{ / thickness [mm].}$$

Hydrophilic Properties

The "hydrophilic properties" of a nonwoven web may be measured using the "Strike Through Time" test. The test used herein is the EDANA standard test WSP 70.3-2005 The lower the value, the more hydrophilic is the web.

Opacity

The opacity of a material is the degree to which light is blocked by that material. A higher opacity value indicates a higher degree of light block by the material. Opacity may be measured using a 0° illumination / 45° detection, circumferential optical geometry, spectrophotometer with a computer interface such as the HunterLab LabScan XE running Universal Software (available from Hunter Associates Laboratory Inc., Reston, VA). Instrument calibration and measurements are made using the standard white and black calibration plates provided by the vendor. All testing is performed in a room maintained at about 23 ± 2 °C and about 50 ± 2 % relative humidity.

Configure the spectrophotometer for the XYZ color scale, D65 illuminant, 10° standard observer, with UV filter set to nominal. Standardize the instrument according to the manufacturer's procedures using the 1.20 inch port size and 1.00 inch area view. After calibration, set the software to the Y opacity procedure.

To obtain the specimen, lay the sample flat on a bench, body facing surface downward, and measure the total longitudinal length of the article. Note a site 33% of the total length from the front waist of the article along the longitudinal axis and a second site, 33% of the total length from the back waist of the article. Carefully remove the backsheet laminate, consisting of both the film and nonwoven web, from the garment-facing side of the article. A cryogenic spray, such as Cyto-Freeze (obtained from Control Company, Houston, TX), may be used to separate the backsheet laminate from the article. Cut a piece 50.8 mm by 50.8 mm centered at each site identified above. Precondition samples at about 23 °C ± 2 C° and about 50% ± 2% relative humidity for 2 hours prior to testing.

Place the specimen over the measurement port. The specimen should completely cover the port with the surface corresponding to the garment-facing surface of the article directed toward the port. Cover the specimen with the white standard plate. Take a reading, then remove the white tile and replace it with black standard tile without moving the specimen. Obtain a second reading, and calculate the opacity as follows:

$$\text{Opacity} = Y \text{ value}_{(\text{black backing})} \ / \ Y \text{ value}_{(\text{white backing})} \ \text{x} \ 100$$

A total of five identical articles are analyzed and their opacity results recorded. Calculate and report the average opacity and standard deviation for the 10 backsheet laminate measurements to the nearest 0.01%.

Using the same specimens as above, remove the nonwoven web from the film layer for analysis. The cryogenic spray can once again be employed. Precondition samples at about 23 °C ± 2 C° and about 50% ± 2% relative humidity for 2 hours prior to testing. In like fashion, analyze the nonwoven web layer following the above procedure. Calculate and report the average opacity and standard deviation for the 10 nonwoven web measurements to the nearest 0.01%.

Bond Shape Measurement Methods

Area, distance and angle measurements are performed on images generated using a flat bed scanner capable of scanning at a resolution of at least 4800 dpi in reflectance mode (a suitable scanner is the Epson Perfection V750 Pro, Epson, USA). Measurements are performed using ImageJ software (Version 1.43u, National Institutes of Health, USA) and calibrated against a ruler certified by NIST.

Samples of the subject nonwoven web that are 80 mm by 80 mm are used. Precondition the samples at about 23 °C ± 2 C° and about 50% ± 2% relative humidity for 2 hours prior to testing. Identify the machine direction of the nonwoven web and draw a fine line on each sample along the machine direction to enable scanned images to be aligned.

Place the sample to be measured on the flat bed scanner, with the surface bearing the bond impressions or bond shapes facing downward, with the ruler directly adjacent. Placement is such that the dimension corresponding to the machine direction of the nonwoven is parallel to the ruler. A black backing is placed over the specimen and the lid to the scanner is closed. Acquire an image composed of the nonwoven and ruler at 4800 dpi in reflectance mode in 8 bit grayscale and save the file. Open the image file in ImageJ and perform a linear calibration using the imaged ruler.

Unless otherwise stated, dimensional and area measurements are made in triplicate, of three similar bond shapes on each sample for 6 similar samples. The 18 values are averaged and reported.

Not intending to be bound by the specific examples, Figs. 5A through 6B are referenced to illustrate the following dimension measurements. These measurement methods are equally applicable to other bond shapes and repeating bond patterns.

Greatest Measurable Length (L)

The bond shape has a perimeter and a greatest measurable length. Identify a shape length line (e.g. line 104) which intersects the two farthest-most points along the perimeter. Draw a shape length line through these points. With the measuring tool, measure the length along the line segment between these points to the nearest 0.001 mm. For example, the greatest measurable lengths in Figs. 5B and 6B are indicated at L, respectively measured along shape length lines 104.

Greatest Measurable Width (W)

Relative the greatest measurable length, the bond shape has a greatest measurable width measured along a direction perpendicular to the shape length line. Draw two lines, parallel to the shape length line, and tangent to the bond shape perimeter at one or more outermost points that are most distant from the shape length line. These are the shape width lines. With the measuring tool, measure the greatest measurable width between the shape width lines along a line segment perpendicular to the shape length line to the nearest 0.001 mm. For example, the greatest measurable widths in Figs. 5B and 6B are indicated at W, respectively measured between lines 105a and 105b perpendicular to shape length lines 104.

Minimum Passageway Clearance

Any two adjacent bond shapes have minimum passageway clearance, defined as the smallest measurable distance therebetween. Identify the two parallel lines, one tangent to the perimeter of the first shape where it appears closest to the second shape, and one tangent to the perimeter of the second shape where it appears closest to the first shape, that lie closer together than any other such parallel lines that can be identified. The minimum passageway clearance is the distance between the identified parallel lines, measured along a line perpendicular to them.

Camber Height (CH)

If the bond shape has a perimeter with a convex portion, the convex portion has a maximum distance from the shape length line, referred to herein as the camber height. Draw a line that is tangent to the convex portion, and parallel to the shape length line. With the measuring tool, measure the distance between width between this tangent line and the shape length line along a direction perpendicular to the shape length line, to the nearest 0.001 mm. For example, the camber heights of the convex portions in Figs. 5B and 6B are CH, and $CH_a$ and $CH_b$, respectively.

Concavity Depth (D)

If the bond shape has a perimeter with a concave portion, the concave portion has a maximum distance from the facing shape width line. Draw a line that is tangent to the deepest point along the concave portion of the profile, and parallel to the shape length line. This is the shape concavity line. With the measuring tool, measure the distance between shape concavity line and the shape length line along a direction perpendicular to the shape length line to the nearest 0.001 mm. For example, the concavity depths of the concave portions in Figs. 5B and 6B are D, and Da and Db, respectively.

Shape Tilt Angle ($\alpha_T$)

The bond shape is rotationally oriented relative the machine direction by shape tilt angle $\alpha_T$. Draw a line in the cross direction, intersecting the shape length line. Draw a line in the machine direction perpendicular to the cross direction line, intersecting both the cross direction line and the shape length line. Using the angle measuring tool, measure the smaller angle between the machine direction line and the shape length line to the nearest 0.1 degree. For example, the angle between lines 108 and 104 in Figure 5B is the shape tilt angle $\alpha_T$.

Pattern Tilt Angle ($\gamma_P$)

The bond shapes may form a pattern that is tilted from the machine direction by the angle $\gamma_P$. Identify a repeating series of bond shapes in a column. Draw a column line that is tangent on one side at the same position on two similar shapes having similar rotational orientations in the column. Draw a line in the machine direction that intersects this column line at an angle, if such a line exists. With the angle measuring tool, measure the smaller angle between the column line and the machine direction line to the nearest 0.1 degree.

Airflow Restriction Ratio

The bond shapes form a pattern that identifies a maximum airflow restriction by the corresponding bonding roller at the nip. Identify a repeating series of bond shapes lying in a row. Draw a line in the cross direction which intersects these bond shapes at the position relative the machine direction where the shapes occupy the greatest proportion of the distance along the cross direction line. It will be appreciated that it may be necessary to take measurements along several cross direction lines to empirically and/or iteratively identify the one along which the bond shapes occupy the greatest proportion of the distance. With the measuring tool, measure the length from the start of the repeating series to the corresponding location at the end of the repeating series (including distances between bonding shapes) to the nearest 0.001 mm. This is the repeat length in the cross direction. With the measuring tool, measure each of the lengths of the line segments on the cross direction line that lie over the bond shapes, to the nearest 0.001 mm. Add the lengths of all of these line segments within the repeat length, and divide the total by the repeat length. Report to the nearest 0.001. This is the airflow restriction ratio. For example, in Figure 5C, the repeat length $w_p$ is measured along the cross direction line 107a. The line segments lying over the bond shapes are $w_1$ through $w_4$. The airflow restriction ration is the sum of lengths $w_1$ through $w_4$ divided by the repeat length $w_p$.

Cross-nip Airflow Angle ($\beta_A$)

The bond pattern may provide an airflow path that has a machine direction vector component. Draw a line in the cross direction. Identify a line that can be drawn that extends past at least eight rows of bond shapes without intersecting a bond shape, if such a line exists. This is the cross-nip airflow line. Extend this line to intersect the cross direction line. Using the angle measurement tool, measure the smaller angle between the cross direction line and the airflow line and report to the nearest 0.1 degree. For example, lines 109 in Figure 5A and 109 in figure 6A are cross-nip airflow lines which intersect the cross direction lines 107 to form the cross-nip airflow angles $\beta_A$.

Bond Area Percentage

Identify a single repeat pattern of bond shapes and areas between them and enlarge the image such that the repeat pattern fills the field of view. In ImageJ, draw a rectangle that circumscribes the repeat pattern. Calculate area of the rectangle and record to the nearest 0.001 mm$^2$. Next, with the area tool, trace the individual bond shapes or portions thereof that are entirely within the repeat pattern/rectangle and calculate and add the areas of all bond shapes or portions thereof that are within the repeat pattern/rectangle. Record to the nearest 0.001 mm$^2$. Calculate as follows:

$$\text{Bond Area \%} = (\text{Sum of areas of bond shapes within repeat pattern}) / (\text{total area of repeat pattern}) \times 100\%$$

Repeat for a total of three non-adjacent regions randomly selected across the sample. Record as Percent Bond Area to the nearest 0.01%. Calculate the average and standard deviation of all 18 of the bond area percentage measurements and report to the nearest 0.01 %.

Average Individual Bond Area

Enlarge the image of a region of the sample such that edges of a bond shape can be identified. With the area tool, manually trace the perimeter of a bond. Calculate and record the area to the nearest 0.001 mm$^2$. Repeat for a total of five non-adjacent bonds randomly selected across the total sample. Measurements are made on each sample. A total of six samples are measured. Calculate the average and standard deviation of all 30 bond area measurements and report to the nearest 0.001 mm$^2$.

**Claims**

1. An article of manufacture (10), the article being an absorbent article, having as a component a section of a nonwoven web (21) having a macroscopic surface approximating a plane, a machine direction (MD) and a cross direction (CD) perpendicular to the machine direction, the nonwoven web formed predominately of polymeric fibers and comprising a series of one or more consolidating bonds impressed on the surface, the one or more consolidating bonds having at least one bond shape (100); wherein the series is repeated to form a pattern of consolidating bonds; wherein the series is repeated in at least four rows (110) extending predominately in the cross direction, and the series is repeated in at least four columns (112) extending predominately in the machine direction; and wherein the bond shape has a perimeter with a greatest measurable length (L) and a greatest measurable width (W), and the perimeter:

   has a convex portion (102a, 102b);
   has an aspect ratio of the greatest measurable length (L) to the greatest measurable width (W) of at least 2.0;
   is oriented such that a line intersecting the perimeter along which the greatest measurable length exists intersects an axis lying on the surface along the machine direction to form a smaller angle ($\alpha_X$) of between 1 degree and 40 degrees; and
   a first of the four rows overlaps a second of the four rows by 15 percent to 25 percent.

2. The article of either of claim 1 wherein the pattern has a nip airflow restriction ratio of 0.40 or less.

3. The article of any of the preceding claims having a bond area percentage of from 8 to 20 percent.

4. The article of any of claims 1 or 2 having a bond area percentage of from 10 to 18 percent.

5. The article of any of claims 1 or 2 having a bond area percentage of from 12 to 16 percent.

6. The article of any of the preceding claims wherein the pattern has a pattern tilt angle ($\gamma_P$) of from greater than 0, to 5 degrees.

7. The article of any of claims 1 to 5 wherein the pattern has a pattern tilt angle ($\gamma_P$) of from 0.5 to 4.5 degrees.

8. The article of any of the preceding claims wherein the pattern has a bond number density from 1.5 bonds/cm$^2$ to 6.0 bonds/ cm$^2$.

9. The article of any of claims 1 to 7 wherein the pattern has a bond number density from 2.0 bonding protrusions/cm$^2$ to 5.0 bonding protrusions/cm$^2$.

10. The article of any of claims 1 to 7 wherein the pattern has a bond number density from 2.5 bonding protrusions/cm$^2$ to 4.0 bonding protrusions/cm$^2$.

11. The article of any of the preceding claims wherein the bonds have an average individual bond surface area of from 2.5 mm$^2$ to 8.0 mm$^2$.

12. The article of any of claims 1 to 10 wherein the bonds have an average individual bond surface area of from 3.0 mm$^2$ to 6.0 mm$^2$.

13. The article of any of claims 1 to 10 wherein the bonds have an average individual bond surface area of from 3.5 mm$^2$ to 5.5 mm$^2$.

14. The article of any of the preceding claims wherein the bond shape (100) describes an "S" shape.

15. The article of any of the preceding claims wherein the bond shape perimeter has a concave portion (103 a, 103b).

**Patentansprüche**

1. Herstellungsartikel (10), wobei der Artikel ein Absorptionsartikel ist, der als eine Komponente einen Abschnitt einer Vliesbahn (21) mit einer sich einer Ebene annähernden makroskopischen Oberfläche, einer Maschinenlaufrichtung (MD) und einer senkrecht zu der Maschinenlaufrichtung verlaufenden Querrichtung (CD) aufweist, wobei die Vliesbahn vorwiegend aus Polymerfasern gebildet ist und eine Reihe von einer oder mehreren verfestigenden Bindungen umfasst, die auf die Oberfläche aufgeprägt sind, wobei die eine oder die mehreren verfestigenden Bindungen mindestens eine Bindungsform (100) aufweisen; wobei die Reihe wiederholt wird, um ein Muster von verfestigenden Bindungen zu bilden; wobei die Reihe in mindestens vier Zeilen (110) wiederholt wird, die sich vorwiegend in Querrichtung erstrecken, und die Reihe in mindestens vier Spalten (112) wiederholt wird, die sich vorwiegend in Maschinenlaufrichtung erstrecken; und wobei die Bindungsform einen Umfang mit einer größten messbaren Länge (L) und einer größten messbaren Breite (W) aufweist und der Umfang:

   einen konvexen Abschnitt (102a, 102b) aufweist;
   ein Seitenverhältnis der größten messbaren Länge (L) zur größten messbaren Breite (W) von mindestens 2,0 aufweist;
   derart ausgerichtet ist, dass eine Linie, die den Umfang schneidet, entlang dem die größte messbare Länge existiert, eine Achse schneidet, die entlang der Maschinenrichtung auf der Oberfläche liegt, um einen kleineren Winkel ($\alpha_X$) zwischen 1 Grad und 40 Grad zu bilden; und
   eine erste der vier Reihen eine zweite der vier Reihen um 15 Prozent bis 25 Prozent überlappt.

2. Artikel nach Anspruch 1, wobei das Muster ein Spaltluftstrombegrenzungsverhältnis von 0,40 oder weniger aufweist.

**3.** Artikel nach einem der vorstehenden Ansprüche mit einem Prozentsatz an Bindungsfläche von 8 bis 20 Prozent.

**4.** Artikel nach einem der Ansprüche 1 oder 2 mit einem Prozentsatz an Bindungsfläche von 10 bis 18 Prozent.

**5.** Artikel nach einem der Ansprüche 1 oder 2 mit einem Prozentsatz an Bindungsfläche von 12 bis 16 Prozent.

**6.** Artikel nach einem der vorstehenden Ansprüche, wobei das Muster einen Musterneigungswinkel ($\gamma_P$) von mehr als 0 bis 5 Grad aufweist.

**7.** Artikel nach einem der Ansprüche 1 bis 5, wobei das Muster einen Musterneigungswinkel ($\gamma_P$) von 0,5 bis 4,5 Grad aufweist.

**8.** Artikel nach einem der vorstehenden Ansprüche, wobei das Muster eine Bindungszahldichte von 1,5 Bindungen/cm$^2$ bis 6,0 Bindungen/cm$^2$ besitzt.

**9.** Artikel nach einem der Ansprüche 1 bis 7, wobei das Muster eine Bindungszahldichte von 2,0 Bindungsvorsprüngen/cm$^2$ bis 5,0 Bindungsvorsprüngen/cm$^2$ aufweist.

**10.** Artikel nach einem der Ansprüche 1 bis 7, wobei das Muster eine Bindungszahldichte von 2,5 Bindungsvorsprüngen/cm$^2$ bis 4,0 Bindungsvorsprüngen/cm$^2$ aufweist.

**11.** Artikel nach einem der vorstehenden Ansprüche, wobei der Artikel einen durchschnittlichen individuellen Bindungsoberflächenbereich von 2,5 mm$^2$/g bis 8,0 mm$^2$/g aufweist.

**12.** Artikel nach einem der Ansprüche 1 bis 10, wobei die Bindungen einen durchschnittlichen individuellen Bindungsoberflächenbereich von 3,0 mm$^2$ bis 6,0 mm$^2$ aufweisen.

**13.** Artikel nach einem der Ansprüche 1 bis 10, wobei die Bindungen einen durchschnittlichen individuellen Bindungsoberflächenbereich von 3,5 mm$^2$ bis 5,5 mm$^2$ aufweisen.

**14.** Artikel nach einem der vorstehenden Ansprüche, wobei die Bindungsform (100) eine "S" -Form beschreibt.

**15.** Artikel nach einem der vorstehenden Ansprüche, wobei der Umfang der Bindungsform einen konkaven Abschnitt (103 a, 103b) aufweist.

**Revendications**

**1.** Article manufacturé (10), l'article étant un article absorbant, ayant en tant que composant une section d'une nappe non tissée (21) ayant une surface macroscopique se rapprochant d'un plan, une direction machine (MD) et une direction croisée (CD) perpendiculaire à la direction machine, la nappe non tissée formée principalement de fibres polymères et comprenant une série d'une ou plusieurs liaisons de consolidation imprimées sur la surface, la ou les liaisons de consolidation ayant au moins une forme de liaison (100) ; dans lequel la série est répétée pour former un motif de liaisons de consolidation ; dans lequel la série est répétée dans au moins quatre rangées (110) s'étendant principalement dans la direction croisée, et la série est répétée dans au moins quatre colonnes (112) s'étendant principalement dans la direction machine ; et dans lequel la forme de liaison a un périmètre avec une plus grande longueur mesurable (L) et une plus grande largeur mesurable (W), et le périmètre :

a une partie convexe (102a, 102b) ;
a un rapport d'aspect de la plus grande longueur mesurable (L) à la plus grande largeur mesurable (W) d'au moins 2,0 ;
est orienté de telle sorte qu'une ligne croisant le périmètre le long duquel la plus grande longueur mesurable existe croise un axe se trouvant sur la surface le long de la direction machine pour former un angle plus petit ($\alpha_X$) compris entre 1 degré et 40 degrés ; et
une première des quatre rangées chevauche une deuxième des quatre rangées à raison de 15 pour cent à 25 pour cent.

**2.** Article selon la revendication 1 dans lequel le motif a un rapport de restriction de flux d'air de ligne de contact de 0,40 ou moins.

**3.** Article selon l'une quelconque des revendications précédentes ayant un pourcentage de surface de liaison allant de 8 à 20 pour cent.

**4.** Article selon l'une quelconque des revendications 1 ou 2 ayant un pourcentage de surface de liaison allant de 10 à 18 pour cent.

**5.** Article selon l'une quelconque des revendications 1 ou 2 ayant un pourcentage de surface de liaison allant de 12 à 16 pour cent.

**6.** Article selon l'une quelconque des revendications précédentes dans lequel le motif a un angle d'inclinaison de motif ($\gamma_P$) allant de plus de 0, à 5 degrés.

**7.** Article selon l'une quelconque des revendications 1 à 5 dans lequel le motif a un angle d'inclinaison de motif ($\gamma_P$) allant de 0,5 à 4,5 degrés.

**8.** Article selon l'une quelconque des revendications précédentes dans lequel le motif a une densité en nombre de liaisons allant de 1,5 liaison/cm$^2$ à 6,0 liaisons/cm$^2$.

**9.** Article selon l'une quelconque des revendications 1 à 7 dans lequel le motif a une densité en nombre de liaisons allant de 2,0 saillies de liaison/cm$^2$ à 5,0 saillies de liaison/cm$^2$.

**10.** Article selon l'une quelconque des revendications 1 à 7 dans lequel le motif a une densité en nombre de liaisons allant de 2,5 saillies de liaison/cm$^2$ à 4,0 saillies de liaison/cm$^2$.

**11.** Article selon l'une quelconque des revendications précédentes dans lequel les liaisons ont une superficie moyenne de liaison individuelle allant de 2,5 mm$^2$ à 8,0 mm$^2$.

**12.** Article selon l'une quelconque des revendications 1 à 10 dans lequel les liaisons ont une superficie moyenne de liaison individuelle allant de 3,0 mm$^2$ à 6,0 mm$^2$.

**13.** Article selon l'une quelconque des revendications 1 à 10 dans lequel les liaisons ont une superficie moyenne de liaison individuelle allant de 3,5 mm$^2$ à 5,5 mm$^2$.

**14.** Article selon l'une quelconque des revendications précédentes dans lequel la forme de liaison (100) décrit une forme en « S ».

**15.** Article selon l'une quelconque des revendications précédentes dans lequel le périmètre de forme de liaison a une partie concave (103 a, 103b).

Fig. 1A

Fig. 1B

Fig. 2A

Fig. 2B

Fig. 3

Fig. 4A

Fig. 4B

Fig. 5A

Fig. 5B

Fig. 5C

Fig. 5D

Fig. 5E

**REFERENCES CITED IN THE DESCRIPTION**

**Patent documents cited in the description**

- US 20130253461 A1
- WO 2014022362 A
- US 5246433 A, Hasse
- US 5569234 A, Buell
- US 6120487 A, Ashton
- US 6120489 A, Johnson
- US 4940464 A, Van Gompel
- US 5092861 A, Nomura
- US 20030233082 A1
- US 5897545 A, Kline
- US 5957908 A, Kline
- US 3860003 A
- US 5151092 A
- US 5554145 A, Roe
- US 6004306 A, Robles
- US 5037416 A, Allen
- US 5269775 A, Freeland
- WO 9516746 A, E. I. DuPont
- US 5571096 A, Dobrin
- US 6645569 B, Cramer
- US 6863933 B, Cramer
- US 7112621 B, Rohrbaugh
- US 20050159720 A
- WO 02064877 A
- US 10758066 B
- US 6632385 B
- US 6803103 B
- US 20060057921
- US 20040131820 A
- US 141122
- US 20040167486 A1
- US 20040162536 A1
- WO 2009060384 A
- US 20080306463
- US 5266392 A
- US 5370764 A